# EUROPEAN PATENT SPECIFICATION

(11) **EP 3 777 875 B1**
(45) Date of publication and mention of the grant of the patent: **03.12.2025**
(21) Application number: 20172555.3
(22) Date of filing: 06.03.2014
(51) Int. Cl.: A61K 38/00, A61M 5/14, A61M 5/00, A61K 9/00, A61J 1/05, A61M 5/28, A61K 9/08, A61K 47/42, A61L 24/00, A61L 24/10, B65D 75/36, A61B 17/00, A61M 5/34, A61M 5/31

(54) **SURGICAL METHODS EMPLOYING PURIFIED AMPHIPHILIC PEPTIDE COMPOSITIONS**
CHIRURGISCHE VERFAHREN UNTER VERWENDUNG AUFGEREINIGTER AMPHIPHILER PEPTIDZUSAMMENSETZUNGEN
PROCÉDÉS CHIRURGICAUX EMPLOYANT DES COMPOSITIONS DE PEPTIDES AMPHIPHILES PURIFIÉS

(30) Priority: 06.03.2013 US 201361773359 P
(43) Date of publication of application: 17.02.2021
(62) Divisional of application: 14759745.4
(73) Proprietor: 3-D Matrix, Ltd., Tokyo 102-0083 (JP)
(72) Inventor: NOHARA, Masahiro, Tokyo 102-0083 (JP); KOBAYASHI, Satoru, Chigasaki, Kanagawa 253-0085 (JP); MATSUDA, Noriaki, Kita-ku, Tokyo 114-0014 (JP)
(74) Representative: EIP

(56) References cited:
- EP-A1- 2 345 433
- WO-A1-2006/116524
- WO-A1-2012/008967
- WO-A2-2013/030673

## Description

### CROSS REFERENCE TO RELATED APPLICATIONS

This application claims benefit under 35 U.S.C. § 119(e) of U.S. provisional patent application serial no. 61/773,359, filed March 6, 2013.

### BACKGROUND

Surgical procedures are performed to correct a variety of medical problems encountered by patients. Typically, an incision is made to access a surgical site within the body of a patient. Blood vessels may be clamped to prevent and/or minimize bleeding; retractors may be employed to expose the surgical site or allow it to remain open thereby permitting a surgeon to perform one or more tasks associated with the procedure. Depending on the work to be performed, several incisions and/or dissections may be necessary in order to penetrate to the surgical site. For example, to gain access to a location in the abdomen, it may be necessary to dissect skin, subcutaneous tissue, muscle layers and/or peritoneum. As is the case in some surgical procedures, it may also be necessary for a surgeon to cut into bone. For example, some surgical procedures may involve cutting the skull to gain access to the brain, or cutting the chest to gain access to the heart. Bleeding can and typically does occur at multiple points in the performance of any or all of these procedures.

Some bleeding during surgery is to be expected. However, extensive bleeding (i.e., beyond what is typically encountered in a given surgical procedure) can be dangerous, even life-threatening. In some cases, severe bleeding may cause a surgical procedure to be terminated. In some cases, a transfusion may be necessary. Blood or blood expanders are typically employed during a surgical procedure to compensate for blood loss. In some cases, steps taken to address blood loss can add considerable time to a surgical procedure and/or lead to longer recovery times for patients.

The standard of care for controlling bleeding during surgical procedures includes the use of synthetic products, materials derived from animals, or human blood components that are locally administered in an as needed manner or by established methodology. Such products and materials are primarily composed of tissue-building proteins are well suited for surgical application as they are biocompatible and demonstrate effectiveness. However, they are not without limitation. For example, these products can present a risk of infection through the presence of infection substances, e.g., viruses. Further, animal-derived products present their own risks in that they can trigger untoward immune responses, potentially including anaphylactic shock, when the patient's body reacts to foreign antigens in the products. WO 2006/116524 A1 discloses a liquid composition for use in surgical procedure to stop bleeding wherein composition comprises a peptide having multiple RADA subunits, such as RADA16.

The present invention provides, among other things, improved surgical procedures that, for example, employ materials that are safer and more effective in controlling and arresting bleeding encountered by surgeons while performing surgical tasks. The present invention also provides surgical procedures that are performed in shorter time and/or involve less bleeding than typically occurs in standard procedures.

### SUMMARY

The present invention provides a composition, comprising:a peptide solution comprising about 1% to about 3% (w/v) of a peptide, wherein the peptide has an amino acid sequence of n-RADARADARADARADA-c; and wherein the solution is characterized by an ability to transition between two states: an ungelled state adopted when one or more particular ions is substantially absent, and a gelled state adopted when the one or more ions is present at or above a threshold level, wherein the one or more ions is or becomes present,for use in a method of preventing post-operative bleeding after a surgical procedure performed on a human patient comprising application of the composition to a bleeding surgical site,wherein the patient is dosed with an anticoagulant.

In some embodiments, one or more ions are selected from potassium (K⁺) and sodium (Na⁺). In some embodiments, one or more ions are potassium (K⁺) and sodium (Na⁺). In various embodiments, a threshold level is characterized by physiological conditions present within a surgical site of a patient or subject. In some embodiments, a threshold level is provided by contact with bodily fluids, blood, tissues and/or a combination thereof within the surgical site of a patient or subject.

In various embodiments, patients or subjects are human or non-human. In some certain embodiments, non-human subjects include mammals. In some certain embodiments, mammals include rodents (e.g., mice or rats), dogs, cats, horses, pigs, cattle, sheep, goats, alpacas, bantengs, bison, camels, deer, donkeys, gayals, guinea pigs, llamas, mules, rabbits, reindeer, water buffalo and yaks.

In some embodiments, an intrabody surgical procedure of the present invention is a resection of or at least a portion of the liver. In some embodiments, a resection of the liver in whole or in part is performed. In some embodiments, of the present invention further comprises an improvement of completing the liver resection within a first period of time that is less than four hours (e.g., less than 3.75, 3.50, 3.00, 2.75, 2.00, 1.75, 1.50, or 1.00) and therefore reduced as compared with the standard first period of time absent such improvement, which standard first period of time is within the range of five to six hours (e.g., within the range of about five to about six hours, inclusive; in some embodiments, about 5.0, 5.1, 5.2, 5.3. 5.4, 5.5, 5.6, 5.7, 5.8, 5.9, 6.0 hours).

In some embodiments, the present invention further comprises an improvement of not applying fibrin glue or SURGICEL^{®} or a combination thereof within the site during the first period of time. In some embodiments, the present invention comprises an improvement of applying the composition comprising a solution of peptides in addition to fibrin glue or SURGICEL^{®} or a combination thereof within the site during the first period of time.

In some embodiments, at least one first application is completed prior to any other surgical activity within the site.

In some embodiments, an intrabody surgical procedure of the present invention is a coronary artery bypass.

The patient or subject is dosed with an anti-coagulant prior to surgery.

In some embodiments, an intrabody surgical procedure of the present invention is a coronary artery bypass in which an improvement further comprises completing the surgical procedure within a first period of time that is at least 20 minutes (e.g., at least 21, 22, 23, 24, 25, 26, 27, 28, 29 or 30 minutes) per graft shorter as compared with the standard first period of time absent such improvement; in some certain embodiments, about 20 minutes.

According to the invention a peptide solution of the present invention has a concentration within the range of 1 - 3%, inclusive; in some certain embodiments, about 1.0%, in some embodiments, about 1.5%; in some embodiments, about 2.0%; in some embodiments, about 2.5%; in some embodiments, about 3%.

The peptide of the present invention comprises an amino acid sequence that comprises four RADA repeats (e.g., RADARADARADARADA; SEQ ID NO:4).

In some embodiments, a pre-filled syringe of the present invention is used in a surgical procedure selected from the group consisting of coronary artery bypass graft (CABG), hepatectomy, pure laparoscopic hepatectomy (PLH), endoscopic mucosal resection (EMR), endoscopic sub mucosal dissection (ESD), thoracoscopic partial lung resection, lymph node dissection, open partial nephrectomy, laparoscopic partial nephrectomy, aorta replacement and orthopedic bone surgery.

In some embodiments, a pre-filled syringe of the present invention comprises a non-metal nozzle that is rigid. In some embodiments, a pre-filled syringe of the present invention comprises a non-metal nozzle that is flexible. In some certain embodiments, a non-metal nozzle is flexible such that it is capable for use in an endoscopic surgical procedure. In some certain embodiments, a non-metal nozzle is flexible such that it is capable for use in a laparoscopic surgical procedure.

In some embodiments, a pre-filled syringe of the present invention comprises a peptide solution as described herein in a volume within the range of about 1 - 50 mL (e.g., about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 mL). In some embodiments, a volume within the range of about 1 to about 10 mL, inclusive; in some certain embodiments, about 1, 2, 3, 4, 5, 6, 7, 8, 9 or 10 mL. In some embodiments, about 1 mL. In some embodiments, about 3 mL. In some embodiments, about 5 mL; in some embodiments, about 10 mL. In some embodiments, a volume within the range of about 20 mL to about 30 mL. In some embodiments, a volume within a range of about 30 mL to about 40 mL. In some embodiments, a volume within a range of about 40 mL to about 50 mL; in some embodiments about 30 mL.

In some embodiments, a kit comprising one or more pre-filled syringes as described herein is provided. In some certain embodiments, a kit comprises one, two, three, four, five, or more pre-filled syringes.

In some embodiments, a pharmaceutical package is provided comprising a pre-filled syringe as described herein and a blister pack specifically formed to accept such pre-filled syringe.

### BRIEF DESCRIPTION OF THE DRAWING

The Drawing included herein, which is comprised of the following Figures, is for illustration purposes only not for limitation.

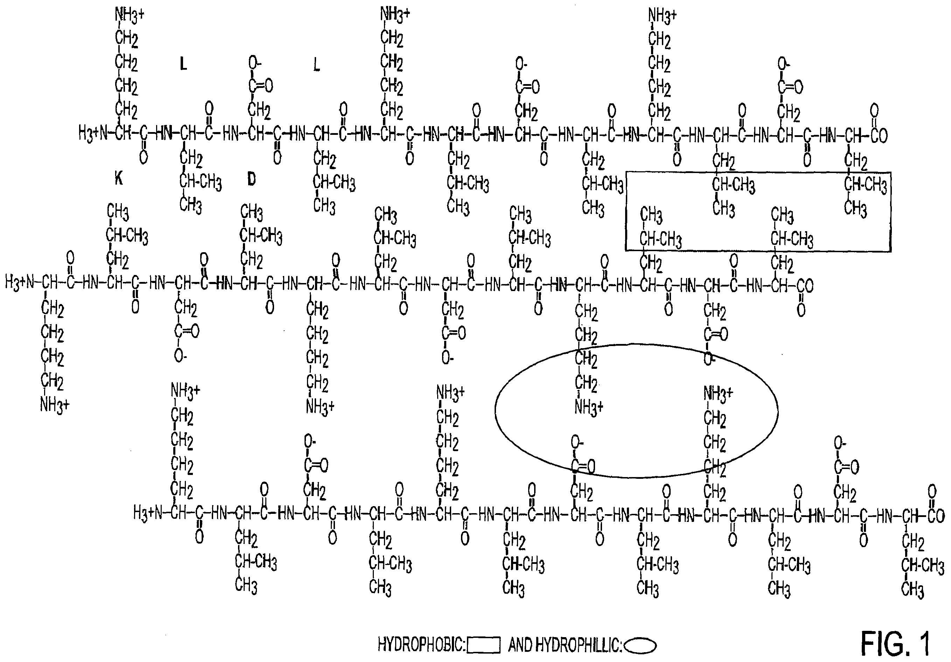

**FIG. 1** is a schematic illustration of the interactions between peptides in the peptide scaffold. Various peptides with amino acid sequences of alternating hydrophobic and hydrophilic residues self-assemble to form a stable scaffold of beta-sheets when exposed to physiologically-equivalent electrolyte solutions (US 5,955,343 and US 5,670,483). The peptide scaffolds are stabilized by numerous interactions between the peptides. For example, the positively charged and negatively charged amino acid side chains from adjacent peptides form complementary ionic pairs, and other hydrophilic residues such as asparagine and glutamine participate in hydrogen-bonding interactions. The hydrophobic groups on adjacent peptides participate in van der Waals interactions. The amino and carbonyl groups on the peptide backbone also participate in intermolecular hydrogen-bonding interactions.

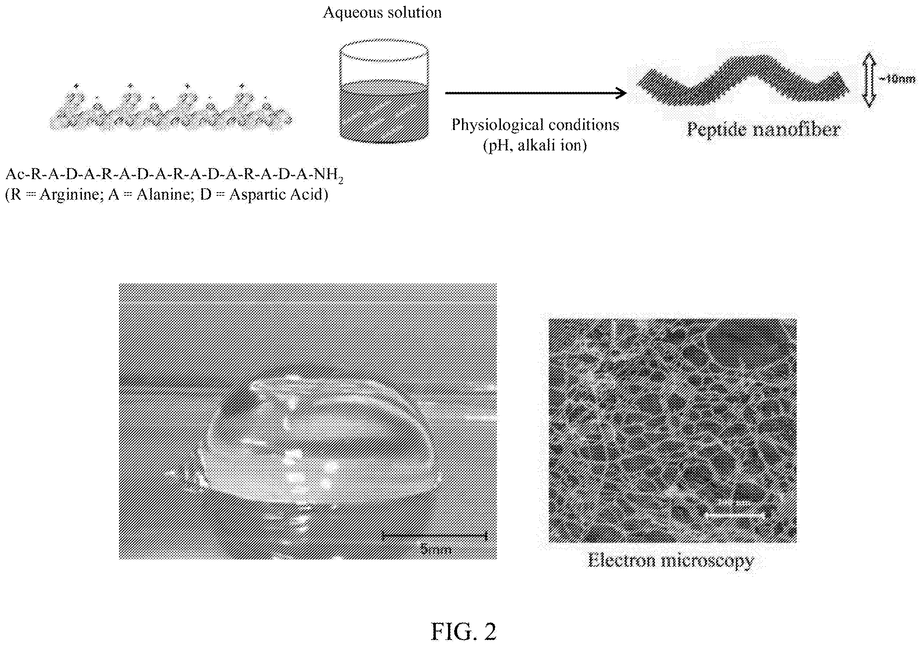

**FIG. 2** shows an illustration of the constituents of a peptide solution and conditions under which the peptide solution forms a fibrous network causing the solution to gel. The peptide chain of RADA repeats is shown (top left) and the resulting formation of a fibrous network after exposure to physiological conditions (top right). An electron micrograph of the fibrous network is shown (bottom right) in addition to the adopted gelled state (bottom left).
**FIG. 3** shows a schematic illustration, not to scale, of the locations of grafts surgically performed on a heart in a typical coronary artery bypass graft (CABG) surgery. The typical steps performed in a CABG surgery are detailed on the right.
**FIG. 4** shows an schematic illustration, not to scale, of the placement of a metal nail plate (left), a gamma nail (middle), and a ender pin (right) in a surgical procedure to repair an intertrochanteric fracture.
**FIG. 5** shows a schematic illustration, not to scale, of the surgical site of a thorascoscopic partial lung resection using a laparoscopy.

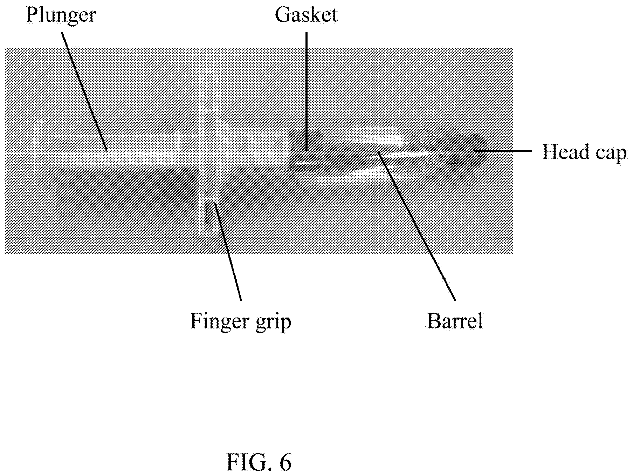

**FIG. 6** shows a picture of a syringe that can be employed for the delivery of a peptide solution to a surgical and/or bleeding site. A plunger, finger grip, gasket, barrel and head cap are labeled.
**FIG. 7** shows a pre-filled syringe with and without a specialized nozzle/cannula attached for delivery of the peptide solution to a surgical and/or bleeding site.
**FIG. 8** shows a pre-filled syringe with and without a specialized connector attached for delivery of the peptide solution to a surgical and/or bleeding site administered through a catheter.
**FIG. 9** shows a pharmaceutical package containing a pre-filled syringe and specialized nozzle/cannula individually supplied in a sterilized blister pack.
**FIG. 10** shows a bar graph of the time during operation to stop bleeding in minutes (x-axis) and the number of application sites (y-axis) according to three exemplary surgical procedures (hepatectomy, angiostomy, endoscopy).

### DETAILED DESCRIPTION OF CERTAIN EMBODIMENTS

The present invention is not limited to particular methods, and experimental conditions described, as such methods and conditions may vary. It is also to be understood that the terminology used herein is for the purpose of describing particular embodiments only, and is not intended to be limiting unless indicated, since the scope of the present invention will be limited only by the appended claims.

Unless stated otherwise, all technical and scientific terms and phrases used herein have the same meaning as commonly understood by one of ordinary skill in the art. Although any methods and materials similar or equivalent to those described herein can be used in the practice or testing of the present invention, the preferred methods and materials are now described.

### DEFINITIONS

As used in this specification and the appended claims, the singular forms "a", "an", and "the" include plural references unless the context clearly dictates otherwise. Thus for example, a reference to "a method" includes one or more methods, and/or steps of the type described herein and/or which will become apparent to those persons skilled in the art upon reading this disclosure and so forth.

The term "carrier" refers to a diluent, adjuvant, excipient, or vehicle with which a composition is administered. Carriers can include sterile liquids, such as, for example, water and oils, including oils of petroleum, animal, vegetable or synthetic origin, such as, for example, peanut oil, soybean oil, mineral oil, sesame oil and the like.

The term "complementary" is used herein to refer to peptides that self-assemble into a scaffold in which ionic or hydrogen bonding interactions occur between hydrophilic residues from adjacent peptides in the scaffold. In many embodiments, as illustrated in Figure 1, each hydrophilic residue in a peptide either interacts (e.g., hydrogen bonds or ionically pairs) with a hydrophilic residue on an adjacent peptide, or is exposed to solvent.

The term "excipient" refers to a non-therapeutic agent added to a pharmaceutical composition to provide a desired consistency or stabilizing effect. Suitable pharmaceutical excipients include, for example, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

The phrase "physiological conditions" refers to conditions of the external or internal mileu that may occur in nature for an organism or cell system. As used herein, physiological conditions are those conditions present within the body of a human or non-human animal, especially those conditions present at and/or within a surgical site. Exemplary physiological conditions are in contrast to conditions in a laboratory setting, which are interpreted to be artificial in comparison. Physiological conditions typically include, e.g., a temperature range of 20 - 40°C, atmospheric pressure of 1, pH of 6 - 8, glucose concentration of 1 - 20 mM, oxygen concentration at atmospheric levels, and gravity as it is encountered on earth.

The term "pure" is used to indicate the extent to which peptide compositions described herein are free of other chemical species, including deletion adducts of the peptide in question and peptides of differing lengths. For example, in some embodiments, a peptide composition is considered to be a "pure" composition of a particular peptide (i.e., of a peptide having a particular amino acid sequence) if substantially all peptides in the composition have amino acid sequences that are identical to the particular sequence, or to a truncation thereof (e.g., a terminal truncation thereof, for example a carboxy-terminal truncation thereof). In some embodiments, at least 90%, 91%, 92%, 93%, 94%, 95%, 96%, 97%, 98%, 99% or more of the peptides in a pure composition of a particular peptide (i.e., of a peptide having a particular amino acid sequence) have amino acid sequences that are identical to the particular sequence, or to a truncation thereof (e.g., a terminal truncation thereof, for example an amino-terminal truncation thereof). In some embodiments, at least 90%, 91%, 92%, 93%, 94%, 95%, 96%, 97%, 98%, 99% or more of the peptides in a pure composition of a particular peptide (i.e., of a peptide having a particular amino acid sequence) are full length.

By the phrase "therapeutically effective amount" is meant an amount that produces the desired effect for which it is administered. In some embodiments, the term refers to an amount that is sufficient, when administered to a population suffering from or susceptible to a disease, disorder, and/or condition in accordance with a therapeutic dosing regimen, to treat the disease, disorder, and/or condition. In some embodiments, a therapeutically effective amount is one that reduces the incidence and/or severity of, and/or delays onset of, one or more symptoms of the disease, disorder, and/or condition. Those of ordinary skill in the art will appreciate that the term "therapeutically effective amount" does not in fact require successful treatment be achieved in a particular individual. Rather, a therapeutically effective amount may be that amount that provides a particular desired pharmacological response in a significant number of subjects when administered to patients in need of such treatment. In some embodiments, reference to a therapeutically effective amount may be a reference to an amount as measured in one or more specific tissues (e.g., a tissue affected by the disease, disorder or condition) or fluids (e.g., blood, saliva, serum, sweat, tears, urine, etc.). Those of ordinary skill in the art will appreciate that, in some embodiments, a therapeutically effective amount of a particular agent or therapy may be formulated and/or administered in a single dose. In some embodiments, a therapeutically effective agent may be formulated and/or administered in a plurality of doses, for example, as part of a dosing regimen.

As used herein, the term "topical" when used to describe application of a composition is intended to describe a situation when the composition is applied to body surfaces such as the skin or mucous membranes as is typically the case in the context of known compositions used in a similar manner, such as, e.g., creams, foams, gels, lotions and ointments. Topical administration is understood to be epicutaneous, meaning that they are applied directly to the skin. Topical administration is also intended to include other formulations that may be applied to the surface of tissues other than the skin, such as eye drops applied to the conjunctiva, or ear drops placed in the ear, or treatment applied to the surface of a tooth. As a route of administration, topical administration are contrasted with enteral (in the digestive tract) and parenteral administration (injected into the circulatory system).

As used herein, the phrase "storage and/or drug delivery system" refers to a system for storing and/or delivering peptide compositions described herein. Exemplary storage and/or delivery systems suitable for peptide compositions described herein are vials, bottles, beakers, bags, syringes, ampules, cartridges, reservoirs or LYO-JECTS^{®}. Storage and/or delivery systems need not be one in the same and can be separate.

As used herein, the term "nozzle" refers to a generally thin, cylindrical object, often with a narrow end and a wide end, which is adapted for fixing onto a delivery device described herein. In some embodiments, the terms "nozzle" and "cannula" are used interchangeably. Nozzles are composed of two connection points or ends, a first connection point or end to connect to a delivery system (e.g. a syringe) and a second connection point which may serve as the point where delivery of pharmaceutical composition is administered or as a point to connect to a secondary device (e.g., a catheter).

The term "bore" is used herein to refer to an opening of a nozzle, cannula and/or catheter that are used in connection with delivery and/or storage systems (e.g., a syringe) containing peptide compositions of the present invention as described herein. Typically, a bore is characterized by various measurements or gauge, e.g., an inner wall diameter thickness, an outer wall diameter and a wall thickness. Exemplary measurements or the gauge of (e.g., diameter, thickness, etc.) a bore of a nozzle, cannula and/or catheter for use in connection with delivery and/or storage systems (e.g., a syringe) containing peptide compositions of the present invention can be found in any needle gauge system (e.g., a French scale or French gauge system, Stubs Iron Wire Gauge system also known as the Birmingham Wire Gauge).

The term "structurally compatible" is used herein to refer to peptides that are capable of maintaining a sufficiently constant intrapeptide distance to allow scaffold formation. In certain embodiments of the invention the variation in intrapeptide distance is less than 4, 3, 2, or 1 angstroms. It is also contemplated that larger variations in intrapeptide distance may not prevent scaffold formation if sufficient stabilizing forces are present. Intrapeptide distance may be calculated based on molecular modeling or based on simplified procedures known in the art (see, for example, U.S. Patent Number 5,670,483). In one exemplary method, intrapeptide distance is calculated by taking the sum of the number of unbranched atoms on the side-chains of each amino acid in a pair. For example, the intrapeptide distance for a lysine-glutamic acid ionic pair is 5+4=9 atoms, and the distance for a glutamine-glutamine hydrogen bonding pair is 4+4=8 atoms. Using a conversion factor of 3 angstroms per atom, the variation in the intrapeptide distance of peptides having lysine-glutamic acid pairs and glutamine-glutamine pairs (e.g., 9 versus 8 atoms) is 3 angstroms.

As used herein, the term "subject" means any mammal, including humans. In certain embodiments of the present invention the subject is an adult, an adolescent or an infant. In some embodiments, terms "individual" or "patient" are used and are intended to be interchangeable with "subject". Also contemplated by the present invention are the administration of the pharmaceutical compositions and/or performance of the methods of treatment in-utero.

### Self-Gelling Peptide Compositions

The present disclosure provides surgical methodologies that are improved through the use of certain sterile self-gelling peptide compositions, as described herein. The present disclosure further provides such compositions specifically prepared for administration during particular surgical procedures. For example, the present disclosure provides specially designed delivery systems (e.g., pre-loaded syringes and/or cannulas) containing such self-gelling peptide compositions.

Compositions, surgical methods and devices disclosed herein provide various improvements to existing methodologies.

Particular exemplary peptides appropriate for use in peptide compositions as described herein include those with sequences reported in U.S. Patents Nos. 5,670,483, and/or 5,955,343, and/or in U.S. Patent Application No. 09/778,200. These peptides have amino acid sequences that consist of alternating hydrophilic and hydrophobic amino acids, and are characterized by an ability to self-assemble in the present of electrolytes (e.g., monovalent cations) into a stable beta-sheet macroscopic structure. Exemplary electrolytes are Na⁺ and K⁺. These peptide chains are self-complementary and structurally compatible. When assembled into the beta-sheet structure, the amino acid side-chains of residues within the peptide partition into one of two faces, a polar face with charged ionic side chains and a nonpolar face with alanines or other hydrophobic groups.

Disclosed peptides have amino acid sequences that consist of alternating positively and negatively charged amino acids. Such peptides are considered to be self-complementary when the positively charged and negatively charged amino acid residues can form complementary ionic pairs. Such peptide chains are referred to as ionic, self-complementary peptides, or Type I self-assembling peptides. If the ionic residues alternate with one positively and one negatively charged residue (-+-+-+-+), the peptide chains are described as "modulus I;" if the ionic residues alternate with two positively and two negatively charged residues (--++--++), the peptide chains are described as "modulus II." Exemplary peptides for use with the present invention include those whose sequences are presented in Table 1 (N/A: not applicable; Asterisk: These peptides form a beta-sheet when incubated in a solution containing NaCl, however they have not been observed to self-assemble to form macroscopic scaffolds).

**TABLE 1**

| Representative Self-Assembling Peptides | | | |
|---|---|---|---|
| Name | Sequence (n-->c) | Modulus | SEQ ID NO: |
| RAD16-I | n-RADARADARADARADA-c | I | 4 |
| RGDA16-I | n-RADARGDARADARGDA-c | I | 5 |
| RADA8-I | n-RADARADA-c | I | 2 |
| RAD16-II | n-RARADADARARADADA-c | II | 6 |
| RAD8-II | n-RARADADA-c | II | 7 |
| EAKA16-I | n-AEAKAEAKAEAKAEAK-c | I | 8 |
| EAKA8-I | n-AEAKAEAK-c | I | 9 |
| RAEAI6-I | n-RAEARAEARAEARAEA-c | I | 10 |
| RAEA8-I | n-RAEARAEA-c | I | 11 |
| KADA16-I | n-KADAKADAKADAKADA-c | I | 12 |
| KADA8-I | n-KADAKADA-c | I | 13 |
| KLD12 | n-KLDLKLDLKLDL-c | | 14 |
| EAH16-II | n-AEAEAHAHAEAEAHAH-c | II | 15 |
| EAH8-II | n-AEAEAHAH-c | II | 16 |
| EFK16-II | n-FEFEFKFKFEFEFKFK-c | II | 17 |
| EFK8-II | n-FEFKFEFK-c | I | 18 |
| KFE12 | n-FKFEFKFEFKFE-c | | 19 |
| KFE8 | n-FKFEFKFE-c | | 20 |
| KFE16 | n-FKFEFKFEFKFEFKFE-c | | 21 |
| KFQ12 | n-FKFQFKFQFKFQ-c | | 22 |
| KIE12 | n-IKIEIKIEIKIE-c | | 23 |
| KVE12 | n-VKVEVKVEVKVE-c | | 24 |
| IEIK9 | n- IEIKIEIKI-c | | 25 |
| IEIK13 | n- IEIKIEIKIEIKI-c | | 26 |
| IEIK17 | n- IEIKIEIKIEIKIEIKI-c | | 27 |
| ELK16-II | n-LELELKLKLELELKLK-c | II | 28 |
| ELK8-II | n-LELELKLK-c | II | 29 |
| EAK16-II | n-AEAEAKAKAEAEAKAK-c | II | 30 |
| EAK12 | n-AEAEAEAEAKAK-c | IV/II | 31 |
| EAK8-II | n-AEAEAKAK-c | II | 32 |
| KAE16-IV | n-KAKAKAKAEAEAEAEA-c | IV | 33 |
| EAK16-IV | n-AEAEAEAEAKAKAKAK-c | IV | 34 |
| RAD16-IV | n-RARARARADADADADA-c | IV | 35 |
| DAR16-IV | n-ADADADADARARARAR-c | IV | 36 |
| DAR16-IV* | n-DADADADARARARARA-c | IV | 37 |
| DAR32-IV | n-(ADADADADARARARAR)²-c | IV | 38 |
| EHK16 | n-HEHEHKHKHEHEHKHK-c | N/A | 39 |
| EHK8-I | n-HEHEHKHK-c | N/A | 40 |
| VE20* | n-VEVEVEVEVEVEVEVEVEVE-c | N/A | 41 |
| RF20* | n-RFRFRFRFRFRFRFRFRFRF-c | N/A | 42 |

Previous studies have demonstrated that charged residues in peptides within Table 1 may be substituted with other residues of the same charge (e.g., substitution of positively charged lysines with positively charged arginines and/or substitution of negatively charged glutamates with negatively charged aspartates) without negatively impacting self-assembly. However, substitution with residues of opposite charge (e.g., substitution of positively charged lysines and/or arginines with negatively charged residues such as aspartate and glutamate) disrupts self-assembly.

Alternatively or additionally, other hydrophilic residues, such as asparagine and glutamine, that form hydrogen-bonds may be incorporated into the peptide chains instead of, or in addition to, charged residues. If the alanines in the peptide chains are changed to more hydrophobic residues, such as leucine, isoleucine, phenylalanine or tyrosine, these peptide chains have a greater tendency to self-assemble and form peptide matrices with enhanced strength. Some peptides that have similar compositions and lengths as the aforementioned peptide chains form alpha-helices and random-coils rather than beta-sheets and do not form macroscopic structures. Thus, in addition to self-complementarity, other factors are likely to be important for the formation of macroscopic scaffolds, such as the chain length, the degree of intermolecular interaction, and the ability to form staggered arrays.

Other self-assembling peptide chains may be generated, for example that have amino acid sequences that differ from that of any self-assembling peptide chains by a single amino acid residue or by multiple amino acid residues. Additionally, the incorporation of specific cell recognition ligands, such as RGD or RAD, into self-assembling peptides may promote the proliferation of cells in the scaffold, and/or may attract cells into the scaffold.

It is disclosed that cysteine can be included in self-assembling peptides, for example to permit formation of disulfide bonds. Alternatively or additionally, residues with aromatic rings may be incorporated into self-assembling peptides, so that cross-links between peptide chains can be generated by exposure to UV light. Table 2 presents representative examples of amino acid sequences of peptides that are susceptible to UV crosslinking. The extent of the cross-linking may be precisely controlled by the predetermined length of exposure to UV light and the predetermined peptide chain concentration. The extent of cross-linking may be determined, for example, by light scattering, gel filtration, or scanning electron microscopy using standard methods. Alternatively or additionally, the extent of cross-linking may be examined by HPLC and/or mass spectrometry analysis of a self-assembled peptide structure after digestion with a protease, such as matrix metalloproteases. The material strength of the scaffold may be determined before and after cross-linking, as described herein.

**TABLE 2**

| Representative Sequences of Cross-Linkable Peptides | | |
|---|---|---|
| Name | Sequence (n-->c) | SEQ ID NO: |
| RGDY16 | RGDYRYDYRYDYRGDY | 43 |
| RGDF16 | RGDFRFDFRFDFRGDF | 44 |
| RGDW16 | RGDWRWDWRWDWRGDW | 45 |
| RADY16 | RADYRYEYRYEYRADY | 46 |
| RADF16 | RADFRFDFRFDFRADF | 47 |
| RADW16 | RADWRWDWRWDWRADW | 48 |

Combinations of any sequences or alterations described herein may be made to any particular self-assembling peptide of interest.

In some embodiments, peptide sequences are selected to achieve a desired level of stiffness and/or elasticity in the structure formed by self-assembly of the peptides. While not wishing to be bound by any theory, low elasticity may help allow cells to migrate into the assembled structure and/or to communicate with one another once resident in the structure.

In some embodiments, peptide sequences are selected to assemble into structures with a low elastic modulus, for example in the range of 1-10 kPa as measured in a standard cone-plate rheometer. Such low values permit scaffold deformation as a result of cell contraction, and this deformation may provide the means for cell-cell communication. In addition, such moduli allow the scaffold to transmit physiological stresses to cells migrating therein, stimulating the cells to produce tissue that is closer in microstructure to native tissue than scar.

Scaffold stiffness can be controlled by a variety of means including, for example, changes in peptide sequence, changes in peptide concentration, changes in peptide length, and combinations thereof. Alternatively or additionally, other methods for increasing stiffness can be used, such as attaching one or more crosslinkable moieties (e.g., biotin) to the peptides (e.g., to the amino terminus, to the carboxy terminus, or to an internal site such as to a side chain) so that they may be cross-linked for example within a self-assembled structure.

In some embodiments, degradation sites such as one or more aggrecan processing sites (e.g., as underlined in Table 3), matrix metalloprotease (MMP) cleavage sites, such as those for collagenase sites, etc. may be introduced into peptides, whether at their amino termini, their carboxy termini, or elsewhere in their sequence the same manner. Peptide structures formed from such degradation-site-containing peptides, alone or in combination with peptides capable of being cross-linked, may be degraded by exposure to appropriate proteases under appropriate conditions (including time of exposure) as understood by those skilled in the art. In some embodiments, the *in vivo* half-life of a structure formed by assembled peptides may be modulated by incorporation of one or more degradation sites into utilized peptides, for example allowing the structure to be enzymatically degraded.

The rate of degradation of peptide structures may be determined, for example, by HPLC, mass spectrometry, and/or NMR analysis of released peptide components. Alternatively or additionally, if radiolabeled peptides are utilized, the amount of released radioactivity may be measured, for example by scintillation counting. For some embodiments, the beta-sheet structure of the assembled peptide chains is degraded sufficiently rapidly that it is not necessary to incorporate cleavage sites into peptides used for assembly.

**TABLE 3**

| Representative Peptide Sequences having Aggrecan Processing Sites | | |
|---|---|---|
| Name | Sequence (N-->C) | SEQ ID NO: |
| REEE | RGDYRYDYTFREEE-GLGSRYDYRGDY | 49 |
| KEEE | RGDYRYDYTFKEEE-GLGSRYDYRGDY | 50 |
| SELE | RGDYRYDYTASELE-GRGTRYDYRGDY | 51 |
| TAQE | RGDYRYDYAPTAQE-AGEGPRYDY-RGDY | 52 |
| ISQE | RGDYRYDYPTISQE-LGQRPRYDYRGDY | 53 |
| VSQE | RGDYRYDYPTVSQE-LGQRPRYDYRGDY | 54 |

In some embodiments, utilized peptides possess an alternating structure of the hydrophobic amino acid alanine (A) and the hydrophilic amino acids arginine (R) and aspartate (D), in which the respective positive and negative charges determine the relative position of the adjoining molecules. Without wishing to be bound by any particular theory, it is proposed that in such embodiments, self-assembly may be completed by hydrophobic bonding between neutral amino acid side chains and hydrogen bonding between peptide backbones. In some such embodiments, utilized peptides have an amino acid sequence that comprises, or in some embodiments consists of, repeats of arginine-alanine-aspartate-alanine (RADA). Disclosed are peptides containing two, three, four or more repeats of RADA (SEQ ID NO:1). According to the invention, the utilized peptides contain four RADA repeats (e.g., have the sequence RADARADARADARADA; SEQ ID NO:4).

The peptides utilized in peptide compositions as described herein are exactly 16 amino acids long.

In some embodiments, peptides utilized in peptide compositions as described herein comprise or consist of natural amino acids; in some embodiments they include one or more non-natural and/or modified amino acids.

In some embodiments, peptides utilized in peptide compositions as described herein comprise or consist of D- amino acids. In some embodiments, peptides utilized in peptide compositions as described herein comprise or consist of L- amino acids. In some embodiments, peptides utilized in peptide compositions as described include both D- and L-amino acids.

In some embodiments, peptides utilized in peptide compositions as described herein are synthesized, for example using standard f-moc chemistry and purified using high pressure liquid chromatography.

In some embodiments, a peptide composition for use in accordance with the present invention is or comprises a bioabsorbable aqueous solution having as its main constituent a peptide. In some embodiments, such a solution is characterized by an ability to transition between two states: an un-gelled state adopted, for example at a particular pH and/or when one or more particular ions is substantially absent, and a gelled state adopted at a particular pH and/or when the one or more ions is present at or above a threshold level.

In some embodiments, transition from un-gelled to gelled state (e.g., via peptide self-assembly) occurs when the peptide solution is exposed to pH in the vicinity of the isoelectric point; in some such embodiments, the isoelectric point is around pH 7. In some embodiments, such transition (e.g., via peptide self-assembly) occurs when the peptide solution is exposed to a pH within a range of about pH 6 to about pH 8, inclusive, for example about 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0; in some embodiments, such pH is within the range of about 6.5 to about 7.5, inclusive; in some embodiments about 6.8 to about 7.2, inclusive; in some embodiments about 7.0.

In some embodiments, transition from un-gelled to gelled state (e.g., via peptide self-assembly) occurs when the peptide solution is exposed to the presence of a low-concentration (e.g., about several millimoles, for example within a range of about 1 millimole to about 10 millimoles, inclusive) of univalent alkali metal ions (e.g., Na⁺, K⁺). In some embodiments, such concentration of univalent alkali metal ions is about 1, 2, 3, 4, 5, 6, 7, 8, 9 or about 10 millimoles. In some embodiments, such concentration is greater than 1 millimole.

In some embodiments, such transition (and/or peptide self-assembly) occurs under physiological conditions (i.e., pH around 7 in the presence of salts such as Na⁺ and K⁺). In some embodiments, such transition (and/or peptide self-assembly) occurs rapidly (e.g., within a time period less than about 5 minutes) upon exposure to appropriate pH and metal ions; in some embodiments, such transition occurs within a time period of about 1 minute to about 5 minutes, inclusive, for example, about 1, 2, 3, 4 or 5 minutes; in some embodiments, such transition occurs within about 5 minutes; in some embodiments such transition occurs within about 4 minutes; in some embodiments, such transition occurs within about 3 minutes; in some embodiments, such transition occurs within about 2 minutes; in some embodiments, such transition occurs within about 1 minute.

In some embodiments, physiological conditions are those present in a body of a subject, e.g., at a surgical site within or on a subject. For example, in some embodiments, physiological conditions can be achieved by the presence of bodily fluids, blood, tissues and/or a combination thereof. In some embodiments, physiological conditions are achieved *in vivo* or *ex vivo* by the addition of a buffer that comprise the ions, e.g., by exogenously adding one or exemplary ions at a level that induces the formation of the gelled state. For example, in some embodiments, peptides may be exposed to appropriate physiological conditions *ex vivo,* for example prior to or during a surgical procedure. In some embodiments, such exposure occurs within a subject's body (e.g., during intrabody surgery), or on a subject's body (e.g., when applied topically, for example, to opening left from a laparoscope or biopsy.

In some embodiments, utilized peptides in solution self-assemble into a structure, for example comprised of a network of fibers, when exposed to an appropriate pH and ion condition. In some embodiments, utilized peptides self-assemble into a network structure that includes fibers and pores. In some embodiments, such fibers have a diameter within the range of about 10 to about 20 nm, inclusive; in some embodiments, such pores have a diameter within the range of about 50 to about 200 nm. In some particular embodiments, a utilized peptide self-assembles into a network structured that resembles the structure of natural collagen (Figure 1).

In some embodiments, peptide compositions for use in accordance with the present invention contain peptides in solution in a concentration within the range of about 1% to about 3%, inclusive.

In various embodiments, the concentration is within the range of 2.0 - 3.0%, 2.25 - 2.75%; in a specific embodiment, the concentration is about 1%; in a specific embodiment, the concentration is about 1.5%; in a specific embodiment, the concentration is about 2%; in a specific embodiment, the concentration is about 2.5%; in a specific embodiment, the concentration is about 3%.

The peptide compositions contain peptides in solution at a concentration within the range of about 1% to about 3%. In some embodiments, peptide compositions for use in accordance with the present invention contains peptides in solution at a concentration of about 1%, 1.5%, 2%, 2.5%, or 3%.

The present invention provides methods of using compositions comprising the peptide solutions described herein, in particular, in methods of performing surgical procedures. In some embodiments, the surgical procedures may be intrabody surgical procedures. In some embodiments, the surgical procedures may be may be superficial or topical.

### Surgical Methods

Peptide compositions described herein may be used in various surgical procedures to control and arrest bleeding encountered by surgeons while performing surgical tasks in a more effective and efficient manner. Exemplary surgical procedures are provided that are performed in shorter time and/or involve less bleeding than typically occurs in standard procedures by use of the peptide compositions described herein.

The present invention provides the insight that peptide compositions as described herein are particularly useful in, and/or provide particular advantages when utilized in certain surgical procedures. For example, among other things, the present invention encompasses the recognition that the peptide compositions described herein provide an effectiveness advantage in arresting bleeding during various surgical procedures. Exemplary advantages are faster completion of one or more surgical tasks during a surgical procedure and, as a result, a decrease in the overall duration of a surgical procedure. In particular, the various examples describe the efficacy and safety of a composition comprising a peptide solution, wherein the peptide comprises an amino acid sequence of RADA repeats; and wherein the solution is characterized by an ability to transition between two states: an ungelled (or aqueous) state adopted when one or more particular ions is substantially absent, and a gelled state adopted when the one or more ions is present at or above a threshold level, wherein the one or more ions is or becomes present in the site (or location) of administration.

In some embodiments, the present invention provides the recognition, among other things, that peptide compositions described herein provide clinical advantages compared to existing materials used in a similar manner for arresting bleeding during surgical procedures.

In some embodiments, the present invention provides the recognition, among other things, that peptide compositions may be manufactured from artificial synthesis without the use of any animal-derived products, negating any risk of infection.

In some embodiments, the present invention provides the recognition that, compared with existing materials, methods of performing a surgical procedure on a subject comprising applying peptide compositions described herein require minimal, or substantially no, preparation and operation, thereby providing an advantage in application.

In some embodiments, the present invention provides the recognition that existing materials (e.g., fibrin glue), in contrast to peptide compositions described herein, are difficult to remove from application sites after hardening. For example, peptide compositions may be washed with saline, allowing for repeated use during surgery.

In some embodiments, the present invention provides the recognition that peptide compositions described herein are colorless and remain transparent once in the gelled state has been adopted during application, thereby maintaining a clear surgical field of view. Such is essential for ascertaining effective control and/or arrest of bleeding from a surgical site.

In some embodiments, the present invention provides the recognition that upon stoppage of bleeding during a surgical procedure or once all or substantially all tasks associated with the a surgical procedure have been completed, excess peptide composition described herein can simply be removed by washing with water. In specific embodiments, after removal of peptide compositions that have been applied to one or more sites on or within a surgical site, secondary bleeding is impeded, inhibited and/or ameliorated by the coagulation system of the subject.

In some embodiments, the present invention provides the recognition that gelation of peptide compositions described herein after contact with blood at or on an application site, rather than solidification within a delivery device, e.g. a nozzle attached to a pre-filled syringe, allows use in specific surgical procedures, e.g., endoscopy and laparoscopy, and thereby eliminates difficulties by using existing materials, which can solidify leading to complications.

In some embodiments, the present invention provides the recognition that peptide compositions described herein provide a contrasting mechanism of action. In certain embodiments, application of peptide compositions described herein to one or more bleeding sites provides a surface pressure on the one or more bleeding sites. Such surface pressure provides normal coagulation to occur beneath the layer of the applied peptide composition once a gelled state is adopted, thereby closing the bleeding site and stopping bleeding. Existing materials require additional manual pressure for compression.

In some embodiments, the present invention provides the recognition that peptide compositions described herein provide a decrease in the time to perform one or more tasks associated with a surgical procedure.

Thus, among other things, the present invention provides improved surgical methods that utilize peptide compositions as described herein. In some embodiments, a provided surgical method is improved relative to a reference or standard of care method in that it is performed in shorter period of time. In some embodiments, a provided surgical method is improved relative to a reference or standard of care method in that recovery of a patient is improved relative to a patient on whom the same surgical method was performed without utilizing peptide compositions described herein.

In some embodiments, peptide compositions described herein are utilized in surgical methods that are performed on the exterior or interior of the body of a subject. In certain embodiments, peptide compositions described herein are utilized in surgical methods that are performed on the vasculature, internal organs and/or bone(s) of a subject.

In some embodiments, peptide compositions described herein are utilized in surgical methods to graft vessels within a surgical site. In certain embodiments, vascular surgical methods comprise bypass surgery (e.g., coronary artery bypass).

In some embodiments, peptide compositions described herein are utilized in surgical methods that are performed to resect or dissect an organ in whole or in part. Virtually an organ may be a candidate in a given surgical procedure, however, without wishing to be bound by theory, exemplary organs may include, e.g., liver, spleen, gall-bladder, pancreas, stomach or lung. In certain embodiments, peptide compositions described herein are utilized in surgical methods that are performed to remove cancerous or otherwise malignant tissue from an organ in whole or in part. In certain embodiments, peptide compositions described herein are utilized in surgical methods that are performed to resect benign tissue of an organ in whole or in part.

In some embodiments, peptide compositions described herein may be utilized in surgical methods performed to repair a fracture of one or more bone(s) of a subject. In certain embodiments, peptide compositions are utilized by injecting into a fracture site of one or more bones in a subject. In certain embodiments, peptide compositions are utilized by applying onto a fracture site of one or more bones in a subject.

In some embodiments, application of peptide composition described herein to a surgical site may vary, e.g., depending upon the application site, patient-specific factors, surgical procedure, application site conditions, route of administration, and the like. When peptide compositions described herein are used for treating various bleeding sites associated with a given surgical procedure, including intrabody surgery in a subject, it is advantageous to administer directly, normally in an amount necessary to arrest bleeding (e.g., a therapeutically effective amount). In some embodiments, the frequency and duration of administering peptide compositions as described herein can be adjusted depending on the severity of the condition(s) or application site.

In some embodiments, peptide compositions described herein utilized in a surgical method are provided in an injectable preparation. Such means for providing peptide compositions for use in arresting bleeding during a surgical procedure is advantageous over existing materials, which may require mixing or otherwise mechanical manipulation on the part of the administrator or surgeon. The injectable preparations may be used for any type of application to a bleeding site of a subject (human or non-human) in need of treatment. A pharmaceutical composition comprising the peptide compositions described herein may be delivered to a bleeding site or surgical site with a syringe and nozzle.

In some embodiments, a subject undergoing a surgical procedure, intrabody or otherwise, may be administered a therapeutically effective amount of peptide compositions as described herein to a bleeding site using a pre-filled syringe. Exemplary techniques include placing a nozzle fixed to the pre-filled syringe in close proximity to one or more bleeding sites as desired.

### Pharmaceutical Compositions

Peptide compositions for use in accordance with the present invention comprise peptides as described herein, optionally together with one or more with suitable carriers, excipients, and/or other agents that are incorporated into formulations; in some embodiments, components of utilized compositions are selected to provide improved transfer, delivery, tolerance, performance, and the like.

In many embodiments, peptide compositions for use in the present invention comprise peptides in aqueous solution (i.e., in a water-based and/or water-miscible carrier). Exemplary aqueous carriers for such compositions include, for example, pharmaceutical grade water, sucrose (e.g., sucrose water), and combinations thereof. In some certain embodiments, peptide compositions for use in the present invention comprise peptides in aqueous solution, wherein the aqueous solution comprises a carrier that is an organic compound that is characterized by an ability to confer solubility and/or bodying effects to the peptides in aqueous solution.

In many embodiments, peptide compositions for use in accordance with the present invention are sterile and/or are prepared aseptically.

In some embodiments, peptide compositions for use in accordance with the present invention, including aqueous formulations, can be stored in an oxygen-deprived environment. Oxygen-deprived environments can be generated, for example, by storing the aqueous solution under an inert gas (e.g., nitrogen or argon).

In some embodiments, peptide compositions for use in accordance with the present invention may be stored in dry form, for example in dry powder form, for example as is achieved by lyophilization.

In some embodiments, peptide compositions for use in accordance with the present invention, specifically including aqueous formulations, are suitably stored at a temperature within the range of about 0°C to about 10°C, inclusive, for example about 0.5°C, 1.0°C, 1.5°C, 2.0°C, 2.5°C, 3.0°C, 3.5°C, 4.0°C, 4.5°C, 5.0°C, 5.5°C, 6.0°C, 6.5°C, 7.0°C, 7.5°C, 8.0°C, 8.5°C, 9.0°C, 9.5°C, or 10.0°C; in some embodiments, such temperature is within the range of about 2.0°C to about 8.0°C, inclusive. In some embodiments, such temperature is above 0°C and lower than 10°C.

In some embodiments, peptide compositions for use in accordance with the present invention are provided in unit dose forms, for example together with a delivery system.

In some embodiments, an appropriate unit dose of a peptide composition in accordance with the present invention, delivers an amount of peptide within the range of about 1% to about 3% (w/v) of peptide, inclusive; for example about 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, or 2.9%. In some embodiments, about 1.0%; in some embodiments, about 1.5%; in some embodiments, about 2.0%; in some embodiments, about 2.5%; in some embodiments, about 3.0%. In some embodiments, an appropriate unit dose of a peptide composition that is a solution is within the range of about 1.0 mL to about 50.0 mL, inclusive, for example about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 mL. In some embodiments, an appropriate unit dose of a peptide composition that is a solution is within the range of about 1.0 mL to about 10 mL. In some embodiments, within a range of about 20 mL to about 30 mL. In some embodiments, within a range of about 30 mL to about 40 mL. In some embodiments, within a range of about 40 mL to about 50 mL. In some embodiments, about 5 mL; in some embodiments, about 10 mL; in some embodiments about 30 mL.

In some embodiments, an appropriate unit dose is about 1 mL to about 5 mL of a 1.0% to 3.0% (w/v) aqueous solution, or about 30 mL of a 1.0% to 3.0% (w/v) aqueous solution.

In some embodiments, a peptide composition as described herein is provided together with (e.g., within) an appropriate storage or delivery container such as for example, a vial, bottle, beaker, bag, syringe, ampule, cartridge, reservoir or LYO-JECT^{®}. In some embodiments, the amount of peptide composition included in such an appropriate storage or delivery container is at least a unit dose of the peptide composition. In some embodiments, the amount is a unit dose, or a multiple thereof. The storage or delivery container may be formed from a variety of materials such as glass or plastic. In some embodiments, peptide compositions for use in accordance with the present invention are provided in a pre-filled syringe, and optionally together with one or more nozzles as described herein for delivery of a peptide solution from such a pre-loaded syringe or other storage container.

Suitable pre-filled syringes include, but are not limited, to, borosilicate glass syringes with baked silicone coating, borosilicate glass syringes with prayed silicone, plastic resin syringes without silicone, or cyclo-olefin-polymer syringes, polypropylene syringes and polyethylene syringes.

In some embodiments, the form of peptide composition that is provided together with (e.g., within) an appropriate storage or delivery container is a solution as described herein; in some embodiments, the form is a dry form as described herein (e.g., a dry powder form).

In various embodiments, peptide compositions for use in accordance with the present invention are suitable for administration to a subject during a surgical procedure. In various embodiments, surgical procedures are performed within the body of a subject, e.g., intrabody. Exemplary intrabody surgical procedures are procedures to correct vascular abnormalities (e.g., a bypass), resection or dissection (e.g., to remove damaged or diseased tissue from an organ in whole or in part), or to repair a damaged organ, tissue or bone (e.g., repair a lacerated spleen, repair a bone fracture, repair torn muscle or ligaments, etc.). In various embodiments, surgical procedures are performed on the exterior of a body of a subject, e.g., topical. Exemplary topical surgical procedures are procedures to repair an opening in the skin of a subject (e.g., sutures to close an opening in the skin made from a puncture or other protrusion). In some embodiments, the subject is a human. In some embodiments, the subject is an non-human animal (e.g., a horse, dog, cat, etc.).

### Storage and/or Delivery Systems

In some embodiments, in addition to providing improved surgical methods as described herein, the present invention provides storage and/or delivery systems particularly adapted for delivery of peptide compositions as described herein In some embodiments, storage systems are separate from delivery systems for peptide compositions described herein. In some embodiments, storage of peptide compositions described herein is provided in delivery systems. For example, peptide compositions described herein may be stored in a delivery system, e.g., a pre-filled syringe, until time for application during a surgical method.

In some embodiments, storage and/or delivery systems as described herein can be utilized in one or more surgical methods. In some embodiments, storage and/or delivery systems as described herein may be utilized in methods for arresting bleeding so as to decrease the duration of a surgical method performed on a subject.

In some embodiments, provided storage and/or delivery systems are particularly adapted for delivery of peptide compositions as described herein to intrabody sites including for example surgical sites. In some embodiments, the present invention provides nozzles and/or cannulas for delivery of compositions such as peptide compositions.

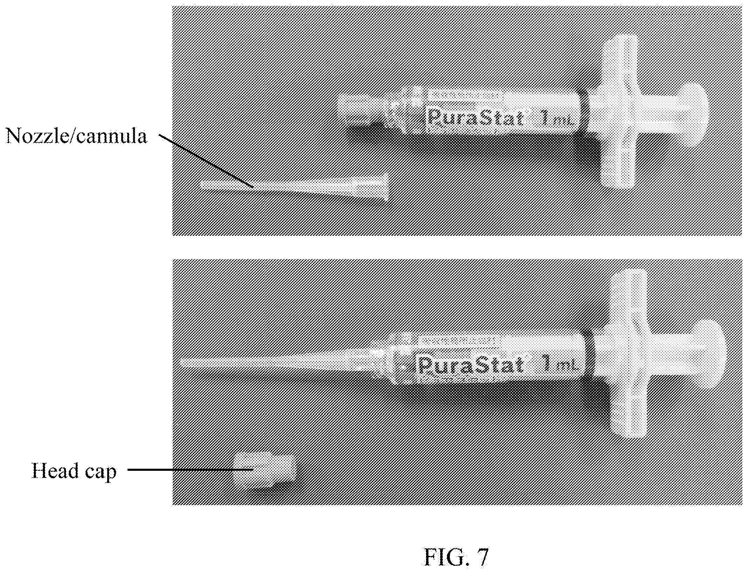

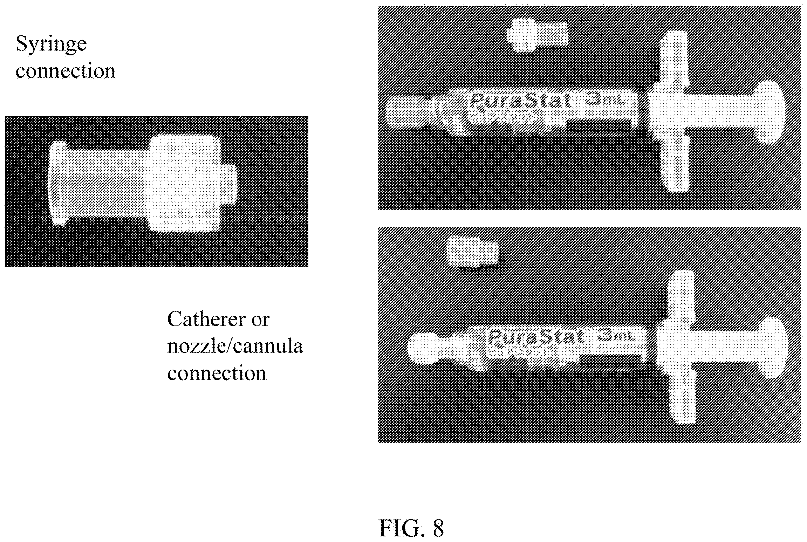

In some embodiments, such nozzles and/or cannulas are adapted for attachment to a syringe or other storage or delivery vessel, which may, for example, be pre-loaded with a composition for delivery. Examples of such nozzles and/or cannulas are depicted in Figures 7 and 8.

In some embodiments, provided nozzles differ from traditional needles in one or more of a variety of features. For example, in some embodiments, exemplary nozzles are made from a non-metal material, in contrast to standard metal needles.

In some embodiments, provided nozzles and/or cannulas are formed from a plastic material (e.g., polypropylene). In certain embodiments, provided nozzles and/or cannulas are formed from a flexible material. In some embodiments, provided nozzles and/or cannulas are formed from a stiff (e.g., non-flexible) material. In some embodiments, provided nozzles and/or cannulas are formed from a material susceptible to sterilization, e.g., by autoclaving.

In some embodiments, provided nozzles and/or cannulas have a blunt end, in contrast to many standard needles, which have a pointed end. For example, standard hypodermic or suture needles, typically have a pointed end, which may be further characterized by a bevel. Exemplary types of bevels include standard, short or true short bevels.

In some embodiments, provided nozzles and/or cannulas have a relatively wide bore as compared with many standard needles. In some embodiments, such nozzles and/or cannulas have an inner bore diameter, an outer bore diameter and a bore wall thickness. For example, in some embodiments, provided nozzles and/or cannulas have an inner bore diameter within the range of about 4.00 mm to about 0.05 mm, inclusive; for example about 4.00 mm, 3.90 mm, 3.80 mm, 3.70 mm, 3.60 mm, 3.50 mm, 3.40 mm, 3.30 mm, 3.20 mm, 3.10 mm, 3.00 mm, 2.90 mm, 2.80 mm, 2.70 mm, 2.60 mm, 2.50 mm, 2.40 mm, 2.30 mm, 2.20 mm, 2.10 mm, 2.00 mm, 1.90 mm, 1.80 mm, 1.70 mm, 1.60 mm, 1.50 mm, 1.40 mm, 1.30 mm, 1.20 mm, 1.10 mm, 1.00 mm, 0.90 mm, 0.80 mm, 0.70 mm, 0.60 mm, 0.50 mm, 0.40 mm, 0.30 mm, 0.20 mm, 0.10 mm, 0.09 mm, 0.08 mm, 0.07 mm, 0.06 mm, or 0.05 mm; in some embodiments, such inner bore diameter is about 3.810 mm, 3.429 mm, 2.997 mm, 2.692 mm, 2.388 mm, 2.159 mm, 1.803 mm, 1.600 mm, 1.372 mm, 1.194 mm, 1.067 mm, 0.838 mm, 0.686 mm, 0.603 mm, 0.514 mm, 0.413 mm, 0.152 mm, 0.337 mm, 0.311 mm, 0.260 mm, 0.127 mm, 0.210 mm, 0.184 mm, 0.159 mm, 0.133 mm, 0.108 mm, or 0.0826 mm; in some embodiments, such an inner bore diameter is within the range of about 1.200 mm to about 0.400 mm, inclusive; in some embodiments, about 1.194 mm, in some embodiments, about 1.067; in some embodiments, about 0.838 mm; in some embodiments, about 0.686 mm; in some embodiments, about 0.603 mm; in some embodiments, about 0.514 mm.

In some embodiments, such an outer bore diameter is within the range of about 5.00 mm to about 0.15 mm, inclusive; for example about 5.00 mm, 4.90 mm, 4.80 mm, 4.70 mm, 4.60 mm, 4.50 mm, 4.40 mm, 4.30 mm, 4.20 mm, 4.10 mm, 4.00 mm, 3.90 mm, 3.80 mm, 3.70 mm, 3.60 mm, 3.50 mm, 3.40 mm, 3.30 mm, 3.20 mm, 3.10 mm, 3.00 mm, 2.90 mm, 2.80 mm, 2.70 mm, 2.60 mm, 2.50 mm, 2.40 mm, 2.30 mm, 2.20 mm, 2.10 mm, 2.00 mm, 1.90 mm, 1.80 mm, 1.70 mm, 1.60 mm, 1.50 mm, 1.40 mm, 1.30 mm, 1.20 mm, 1.10 mm, 1.00 mm, 0.90 mm, 0.80 mm, 0.70 mm, 0.60 mm, 0.50 mm, 0.40 mm, 0.30 mm, 0.20 mm, or 0.10 mm; in some embodiments, such an inner bore diameter is about 4.572 mm, 4.191 mm, 3.759 mm, 3.404 mm, 3.048 mm, 2.769 mm, 2.413 mm, 2.108 mm, 1.829 mm, 1.651 mm, 1.473 mm, 1.270 mm, 1.067 mm, 0.9081 mm, 0.8192 mm, 0.7176 mm, 0.6414 mm, 0.5652 mm, 0.5144 mm, 0.4636 mm, 0.4737 mm, 0.4128 mm, 0.3620 mm, 0.3366 mm, 0.3112 mm, 0.2604 mm, 0.2350 mm, 0.2096 mm, or 0.1842 mm; in some embodiments, such outer bore diameter is within the range of about 1.650 mm to about 0.750 mm, inclusive; in some embodiments, about 1.651; in some embodiments, about 1.473; in some embodiments, about 1.270 mm; in some embodiments, about 1.067 mm; in some embodiments, about 0.9081 mm; in some embodiments, about 0.8192.

In some embodiments, such a bore wall thickness is within the range of about 0.400 mm to about 0.025 mm, inclusive; for example about 0.400 mm, 0.375 mm, 0.350 mm, 0.325 mm, 0.300 mm, 0.0275 mm, 0.250 mm, 0.225 mm, 0.200 mm, 0.175 mm, 0.150 mm, 0.125 mm, 0.100 mm, 0.075, 0.050 mm, or 0.025 mm; in some embodiments, such a bore wall thickness is about 0.381 mm, 0.356 mm, 0.330 mm, 0.305 mm, 0.254 mm, 0.229 mm, 0.203 mm, 0.216 mm, 0.191 mm, 0.1524 mm, 0.2826 mm, 0.1524 mm, 0.1270 mm, 0.1016 mm, 0.1734 mm, 0.1016 mm, 0.0889 mm, 0.0762 mm, 0.0635 mm, or 0.0508 mm; in some embodiments, such a bore wall thickness is within the range of about 0.250 mm to about 0.150 mm; in some embodiments, about 0.229 mm; in some embodiments, about 0.216 mm; in some embodiments, about 0.203 mm; in some embodiments, about 0.191 mm; in some embodiments, about 0.1524 mm.

In some embodiments, provided nozzles and/or cannulas may have a tapered bore. In some embodiments, such provided nozzles and/or cannulas taper substantially evenly between their large and small bore portions. In some embodiments, provided nozzles and/or cannulas taper to a small bore portion at their delivery end, which may for example be a blunt end as described herein.

In some embodiments, provided nozzles and/or cannulas have a length within a range of about 6 inches to about 0.25 inches; inclusive, for example, about 6.0 inches, 5.9 inches, 5.8 inches, 5.7 inches, 5.6 inches, 5.5 inches, 5.4 inches, 5.3 inches, 5.2 inches, 5.1 inches, 5.0 inches, 4.9 inches, 4.8 inches, 4.7 inches, 4.6 inches, 4.5 inches, 4.4 inches, 4.3 inches, 4.2 inches, 4.1 inches, 4.0 inches, 3.9 inches, 3.8 inches, 3.7 inches, 3.6 inches, 3.5 inches, 3.4 inches, 3.3 inches, 3.2 inches, 3.1 inches , 3.0 inches, 2.9 inches, 2.8 inches, 2.7 inches, 2.6 inches, 2.5 inches, 2.4 inches, 2.3 inches, 2.2 inches, 2.1 inches, 2.0 inches, 1.9 inches, 1.8 inches, 1.7 inches, 1.6 inches, 1.5 inches, 1.4 inches, 1.3 inches, 1.2 inches, 1.1 inches, 1.0 inch, 0.9 inches, 0.8 inches, 0.7 inches, 0.6 inches, 0.5 inches, 0.4 inches, 0.3 inches, or 0.2 inches; in some embodiments, about 0.50 inches to about 1.5 inches.

In some embodiments, provided nozzles and/or cannulas are specially adapted for application in a particular surgical procedure. For example, nozzles may be engineered based on type of surgery for which they are used (e.g., endoscopy, laparoscopy, etc.); other factors consideration are size, length and flexibility (e.g., adapted for range of motion, ability to use such that surrounding tissue is not disrupted or damaged), geometry and other formats. Further, as may be appropriate, the addition of or coupling with an optical system and/or light system, thereby allowing for visual confirmation of application to a site otherwise occluded from a surgeon's view. In some embodiments, nozzles and/or cannulas adapted for coupling to a catheter are provided. An example of such adaptor is provided in Figure 8.

In some embodiments, provided nozzles and/or cannulas adapted for coupling to a catheter have a first connection end for connection to a catheter that has a diameter within the range of about one millimeter to about four millimeters. In some embodiments, a diameter within the range of about one millimeter to about 2 millimeters. In certain embodiments, a diameter of about 1.5 millimeters.

In some embodiments, provided nozzles and/or cannulas adapted for coupling to a catheter have a second connection end for connection to a storage and/or delivery device, e.g., a pre-filled syringe, that has a diameter within the range of about four millimeters to about 8 millimeters. In some embodiments, a diameter within the range of about five millimeters to about 7 millimeters. In certain embodiments, a diameter of about five millimeters. In a specific embodiment, a diameter of about 5.21 millimeters. In certain embodiments, a diameter of about seven millimeters. In a specific embodiment, a diameter of about 6.9 millimeters.

Alternatively or additionally, provided nozzles and/or cannulas may be designed in the context of pressure when applying the compositions from a syringe.

In some embodiments, provided nozzles and/or cannulas are reusable, for example, being adapted to be removed from a first storage and/or delivery vehicle (e.g., after delivery of composition from the storage and/or delivery vehicle) and attached to a second (and/or subsequent) storage and/or delivery vehicle. In some embodiments, provided nozzles and/or cannulas are single-use.

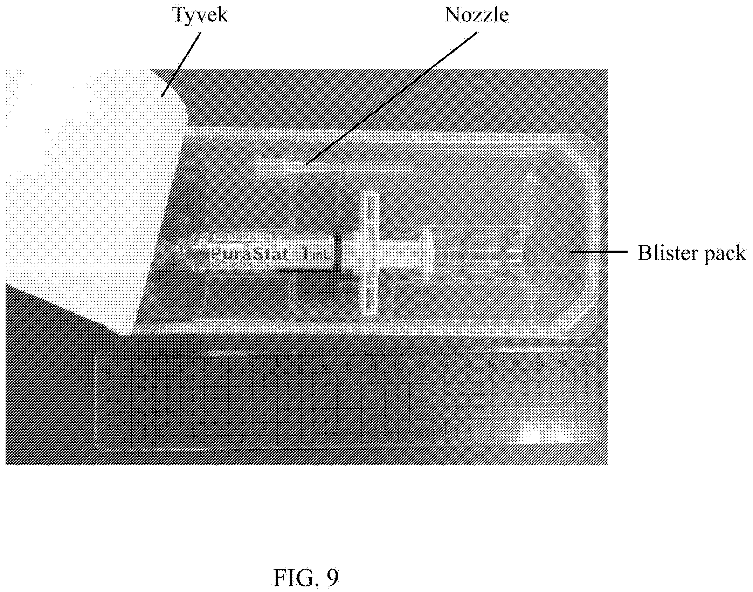

In some embodiments, pharmaceutical packages that contain a storage and/or delivery system described herein are provided. Suitable pharmaceutical packages are sterile and acceptable for use in a surgical setting. Examples of pharmaceutical packages are blister packs, bubble packs or clamshell packages. Pharmaceutically acceptable packages, for example, may be performed packaging and made from a various of materials, such as, e.g., cyclic olefin copolymers ( ), polychlorotrifluoroethylene, or polyvinyl chloride. An example of a pharmaceutical package (e.g., a blister pack) comprising a storage and/or delivery device comprising peptide compositions (e.g., a pre-filled syringe) described herein and a nozzle/cannula is provided in Figure 9.

In some embodiments, a blister pack is provided that contains a cavity or pocket that provides a custom, formable location to accept a storage and/or delivery device comprising peptide compositions described herein. Additionally or optionally, in some embodiments, a blister pack is provided that contains a cavity or pocket that provides a custom, formable location to accept a nozzle or cannula described herein. Additionally or optionally, in some embodiments, a blister pack is provided that contains a cavity or pocket that provides a custom, formable location to accept an adaptor described herein. In various embodiments, individual blister packs contain a cover (e.g., a tyvek sheet) fixed to the cavity or pocket to maintain sterile conditions.

In various embodiments, provided pharmaceutical packages, e.g., blister packs, are sterile. Sterilization (i.e. aseptic processing of storage and/or delivery devices described herein) may be accomplished by methods know in the art and acceptable for pharmaceutical products and/or packages. Examples of sterilization techniques for pharmaceutical packages described herein are pressurized steam, hot air, ionizing radiation (e.g., gama and/or electron beam), and gas (e.g., ethylene oxide or formaldehyde).

In some embodiments, multiple storage and/or delivery devices may be provided in a single pharmaceutical package. For example, a unit or multiple doses provided in pre-filled syringes may be packaged in multiple blister packs, or optionally, in a clamshell-type container suitable for packaging multiple syringes. In some embodiments, pre-filled syringes comprising a unit dose, or multiple doses, are provided in blister packs. In some embodiments, multiple pre-filled syringes are provided in clamshell-type packages and are acceptable for use in a surgical setting (i.e., sterile). Suitable sterilization techniques are employed as described above to ensure sterilized pharmaceutical products are provided in various surgical settings.

### EXAMPLES

The following examples are put forth so as to provide those of ordinary skill in the art with examples of how to make and use the methods and compositions of the invention, and are not intended to limit the scope of the invention. Example 3 is according to the invention as claimed and the other examples are preparatory examples and examples showing other applications of the peptides of the invention. Efforts have been made to ensure accuracy with respect to numbers used (*e.g.,* amounts, temperature, *etc*.) but some experimental deviations are to be expected as is known to one of skill in the art. Unless indicated otherwise, parts are parts by weight, molecular weight is average molecular weight, temperature is in degrees Centigrade, and pressure is at or near atmospheric.

### Example 1. RADA-16 Solution

The inventors have determined that peptide compositions described herein arrest bleeding in a surgical procedure by an entirely different mechanism than that of existing materials used to control and/or stop bleeding during surgery. Typically, coagulation factors are used. However, through the rather rapid gelation under physiological conditions, peptide compositions described herein block the bleeding site without the use of a coagulation factor and can stop bleeding without pharmacological action. The inventors have employed peptide compositions described herein in various surgical procedures on human and non-human subjects and discovered that peptide gels intertwine with blood cells at a given bleeding site at the superficial portion of the blood vessels thereby allowing blood coagulation to occur below the top layer of the gel. Other materials, e.g., fibrin glue, act by activating the blood coagulation system by mobilizing coagulation factors, thereby blocking bleeding from a given site. Exemplary properties of peptide compositions described herein include starting materials of an artificial synthetic peptide and water for injection without including any animal-derived materials. This drastically reduces, if not eliminates altogether, the risk of infection by using peptide compositions described herein in a surgical procedure. Further, because peptide compositions described herein are provided in an aqueous solution it can be supplied in pre-filled syringes and used directly on or within a surgical site. There is no need for special preparation before application, as is typically encountered with other materials, e.g., fibrin glue. Also, a large component of the aqueous solution is water, which allows for repeated use in an almost unlimited manner, unlike other materials, especially adhesive-based materials.

The present Example describes a particular peptide composition, referred to herein as "Composition 1" utilized in various surgical methods as described herein. Composition 1 is a bioabsorbable aqueous solution containing 2 - 2.5% of RADA-16 in water.

Composition 1 is manufactured by preparing peptides consisting of chemically synthesized amino acids using solid-phase synthesis, dissolving the peptides in water for injection, filtering the solution with a bacterial filter (0.2 mm), and filling the resulting filtrate in a sterile manner into a syringe. As such, manufacture is completed without using any animal-derived materials, eliminating any risk of infection by biological materials.

Composition 1 is a clear, colorless liquid and retains this transparent quality upon application to a surgical site upon which the peptide solution adopts a gelled state by the formation of a hydrogel and has the ability to stop bleeding during the performance of a surgical procedure. This transparent quality makes Composition 1 uniquely suited for use in surgical procedures over other materials in terms of its ease of use and ability to maintain a clear surgical field. Composition 1 can be provided in a pre-filled syringe and thus is unique compared to other materials, e.g., fibrin glue, which needs to be prepared and mixed from separate liquids. There is no such requirement with Composition 1 as it is made from peptides and can be completely broken down by washing. The inventors have realized a number of advantages in employing Composition 1 in surgical procedures: virtually unlimited frequency of application, faster and more efficient control and stoppage of bleeding, maintenance of clear surgical field and bleeding site due to transparent quality, easily removed by irrigation, shortens duration of bleeding control measures during surgery, overall shortening of time required to complete surgical procedure, and may improve the rate of patient recovery by contributing to overall decrease in blood loss during surgery.

Cell culture experiments have demonstrated that Composition 1's main constituent peptide (CH₃CO-(Arg-Ala-Asp-Ala)₄-NH₂, see below) does not exhibit bioactivity by acting on the signal transduction system of living organisms (data not shown). A search of the European Molecular Biology Laboratory (EMBL) and Kyoto University's GenomeNet Database Resources for protein sequence motifs for all amino acid sequences in which the main constituent peptide can be generated by cleavage did not reveal any sequences indicating a high degree of homology with known motifs. Once Composition 1 forms a gel, the peptides resist degradation even when exposed to digestive enzymes such as trypsin, α-chymotrypsin, papain, protease K and pronase.

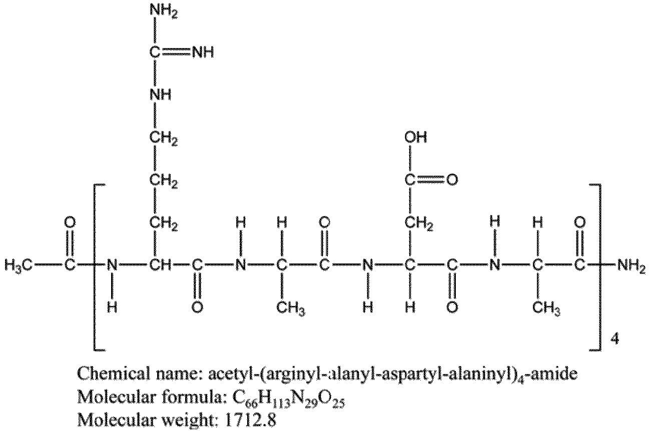

Unlike other measures to control and/or stop bleeding during surgery, e.g., oxidized cellulose or starch-based absorbent topical preparations that stem blood flow by the formation of clots, the mechanism of action of Composition 1 is realized by modification of physical properties upon a change in pH to seal off the bleeding point.

### Example 2. Surgical procedures in non-human animals employing Composition 1

This Example describes certain animal studies based on results of an efficacy validation study of an approved material for controlling bleeding during surgery. The oozing needle hole hemorrhage model of prosthetic vascular graft implantation in a beagle dog was designed to mimic the oozing needle hole hemorrhage from anastomotic sites at the autologous vascular.

All animals received humane care in compliance with the Principles of Laboratory Animal Care formulated by the National Society for Medical Research and the Guide for the Care and Use of Laboratory Animals prepared by the Institute of Laboratory Animal Research (ILAR), published by the National Academies Press (1996).

**Rabbit abdominal aortic puncture.** Laparotomy was performed to expose approximately 10 cm of the abdominal aorta of each rabbit. Heparin sodium (500 IU) was administered intravenously. The bleeding model was established by puncturing the abdominal aorta using an injection needle (23-26G). After bleeding was confirmed, peripheral and central blood flow was stopped with clamps and Composition 1 was immediately applied to the wound site using a syringe. Blood flow was allowed to resume after 1-2 min, and the puncture site was visually inspected for bleeding. Rabbit abdominal aorta used in the present study was fixed in formalin, and vascular cross sections of both Composition 1-treated and untreated sites (control) were used to make pathology specimens that were then observed under a microscope.

The results demonstrated that total cessation of bleeding was observed in all animals following administration with a ≥ 2% peptide concentration of Composition 1, with the exception of one animal treated with a 2% peptide concentration of Composition 1. The structure-less and eosinophilic gelatinized Composition 1 was observed at the vessel puncture site and surface. Further, gelatinized Composition 1 was observed to have formed a coating on the tissue surface that physically occluded the puncture.

**Beagle abdominal aortic graft replacement.** Male beagles (n=2) weighing 13.1 kg and 11.4 kg were employed for an aortic graft replacement surgical procedure using Composition 1. The abdominal aorta was exposed via laparotomy under general anesthesia. Heparin sodium was intravenously infused at 1000 IU. After confirmation that the active coagulation time (ACT) had exceeded 200 seconds, the abdominal aorta was clamped, and an end-to-end graft replacement procedure was performed. Exudative bleeding (an oozing-type bleeding) from the graft anastomosis and needle hole were observed. Composition 1 was applied to the needle hole to evaluate the efficacy and cessation of bleeding.

The results demonstrated that oozing-type bleeding from anastomosis site was stopped, and about 1 min after applying approximately 2 mL of 2.5% Composition 1, stoppage of the anastomotic oozing was confirmed. Further, the oozing-type bleeding from the needle hole was also arrested. The abdominal aorta puncture bleeding model was prepared by piercing the artificial vascular graft with the same-sized 26-G injection needle used on the rabbits (as described above), and spurting of blood was consequently observed. Peripheral and central blood flow was stopped, and approximately 1 mL of 2.5% Composition 1 was applied. Blood flow was allowed to resume after about 1 minute. Complete stoppage of bleeding at the wound site was confirmed. This procedure was repeated three times at three separate sites on the graft. Postoperative observation was performed up to three days.

**Mouse intravenous administration.** Composition 1 forms a gel as soon as it comes in contact with blood from a bleeding site. Through application at a bleeding site, it is possible for an amount of gelatinized Composition 1 to enter the blood stream. [[Note to client: what can we make of this: "There is also an undeniable risk of gelatinized Composition 1 entering the blood stream as a result of erroneous intravascular administration. To evaluate these risks, we carried out tests on mice and rabbits simulating accidental IV administration of Composition 1."]] In this example, the safety of intravenous administration of Composition 1 was demonstrated using mice.

Briefly, gelatinized Composition 1 in suspension was dosed at a concentration that would presumed to have an adverse biological effect. The results demonstrated that death in mouse subjects was observed up to a 40-fold dilution. While direct causes were not determined, it was suspected that death was due to pulmonary embolism. No autopsy was performed on the mice. However, the following abnormal behaviors indicate pulmonary infarction: reduction of spontaneous behavior, squatting position, and accelerated respiration. No deaths were observed at an 80-fold dilution, although abnormal findings (inactivity and tachypnea) were observed. At a 160-fold dilution, no abnormalities were observed.

In a similar experiment, guinea pigs were administered 0.2 mL of Composition 1 in a 160-fold diluted suspension. No abnormal behaviors were observed in any of the animals.

Without wishing to be bound by theory, these results may be extrapolated to humans assuming the following conditions: the subject is an adult weighing 60 kg having a total blood volume of 4.6 L. Assuming this criteria and using the data observed using the mice (body weight of about 40 g and a total blood volume of about 3 mL), the expected amount of Composition 1 gel administered via intravenous injection that would likely cause death in a manner similar to that observed in the mice would be a 4- to 40-fold dilution of approximately 770 mL, or approximately 19.3-193 mL of Composition 1. Such a volume is much larger than that employed in a surgical procedure. As shown by this example, Composition 1 is a safe and effective solution to controlling bleeding in surgical models in various animals.

As shown in this example, application of Composition 1 exhibited efficacy at arresting bleeding in the exemplary animal surgical procedures described above. Further, from the intravenous administration experiments, a single dose of 5 mL Composition 1 via syringe is unlikely to cause pulmonary embolism or other adverse events resulting in death, even in the case of a mistaken administration, directly into a blood vessel.

### Example 3. Coronary Artery Bypass Graft (CABG)

The present Example describes a Coronary Artery Bypass Graft surgery utilizing peptide compositions described herein and, in particular, steps of the surgical method at which peptide compositions described herein can be applied. An exemplary peptide composition provided is Composition 1 (described above).

Coronary Artery Bypass Graft, or CABG, begins with exfoliating the internal thoracic artery or collecting the great saphenous vein, which are to be used as grafts. The internal thoracic artery is anastomosed to the outer area of the heart away from blocked coronary arteries. The collected great saphenous vein is anastomosed to the base of the coronary artery and to outer area in the heart away from lesion (blocked) coronary arteries.

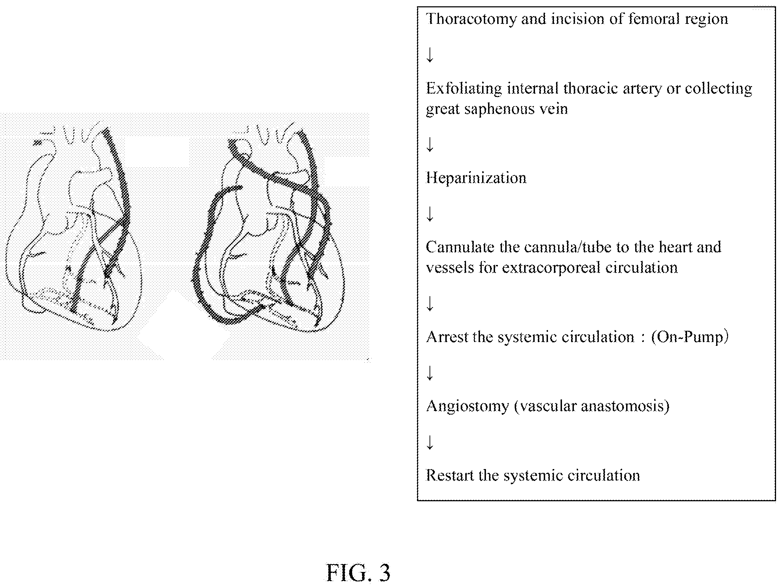

Typically in CABG surgery, the internal thoracic artery as graft would be the first choice because of the historical success rate. Optionally, the great saphenous vein can be used in the event more grafts are necessary. Bleeding can occur during multiple stages of CABG surgery, for example, exfoliating internal thoracic artery or collecting great saphenous vein, which are used as grafts, rebleeding on above areas due to heparinization, anastomosis sites of various heart arteries and grafts, and connection sites of the heart and the cannula (tube) of an oxygenator (Figure 3).

**Bleeding at exfoliation or graft collection sites.** Exfoliation or collection of grafts is performed using standard surgical instruments or an electrosurgical knife. Bleeding from collection sites are usually the result of using an electrosurgical knife and the patient undergoing this surgery is heparinized after this procedure to prevent blood from clotting in preparation for connecting an oxygenator. After heparinization, oozing type of rebleeding frequently occurs from collection sites, at which time an electrosurgical knife is usually applied to stop such rebleeding. This can take extra time that prolongs the CABG procedure and causes damage to tissues. This prolongs the healing process leading to a slower recovery following the procedure. Surgeons desire to minimize overall surgery time required to perform the surgery as well as steps to minimize bleeding during the surgery as the target of the surgery is bypass, not collecting grafts. Existing surgical methods at controlling bleeding are inadequate and are not typically and widely used to control the bleeding during this procedure. Instead, an electrosurgical knife is used and the tissue is burned to stop any bleeding.

In a CABG surgery, Composition 1 can be applied at multiple steps during the procedure to control bleeding and decrease the overall time necessary to complete the surgery. Composition 1 can be applied to a collection site to effectively prevent bleeding. Due to its ease of use, Composition 1 can be applied before and during the collection procedure. Further, it can also be applied before heparinization. By preventing bleeding during the procedure and of rebleeding after heparinization, a surgeon can reduce the total amount of bleeding and shorten the overall time needed to complete the surgery. Further, this time savings is recognized in time required for anesthesia as well.

In one CABG surgery procedure in a patient in need of such surgery, four grafts are required to perform a successful bypass. Time per graft is decreased by 5 to 20 minutes and overall time for the surgery to be performed is decreased by 20 to 80 minutes. This decrease in time is due, in part, to the removal of or reduced need for the electrosurgical knife in the procedure. Further, a decrease risk of infection is observed. As a result in the decreased time for performance of the CABG procedure, a reduction of total hospitalization time is expected, e.g., one 24 hour period. In some cases, where the surgeon encounters difficult rebleeding during the procedure, additional 2 or 3 days of hospitalization are required.

Peptide compositions such as Composition 1 are applied to bleeding sites during and after exfoliating or graft collection in a wide area around the target bleeding sites, and permitted to remain in the area untouched. This allows the solution to form into a gelled state on the target site. Manual manipulation of peptide compositions is not advised, e.g., by rubbing with one's fingers, as this lead to break down of the gel. Peptide compositions remain transparent despite the change in state from a solution to a gel. This unique property allows for the maintenance of a clear surgical field as well as improved and superior control of bleeding from multiple sites during the procedure. The use of a pre-filled syringe containing the solution and a specialized nozzle adapted for use in such a procedure contributes to the reduced time for performing the various steps of the procedure as well as the procedure as a whole. Peptide compositions can be washed from the area at the end of the CABG procedure.

SURGICEL^{®} is made of an oxidized cellulose polymer with a low pH and is used to control post-surgical bleeding by inducing clotting of blood. It has been associated with incidents of neurotoxicity. For example, SURGICEL^{®} is used extensively in oral and maxillofacial surgery to control intrabody arterial bleeds from the inferior alveolar artery. When placed in the mandibular canal with the inferior alveolar nerve exposed there have been reports of neurotoxic effects.

In CABG surgery, SURGICEL^{®} could be applied to bleeding sites, however, surgeons typically prefer an electrosurgical knife. Preferred use electrosurgical is due, in part, to the time needed to use SURGICEL^{®} as it has a cotton-wool or sheet-type property and surgeons have to cut it using tweezers and subsequently apply it to bleeding sites after removing blood. This is a difficult task since it is easy SURGICEL^{®} to stick to tweezers. It is necessary for SURGICEL^{®} to absorb blood to become sticky and thereby control bleeding. Further, applied pressure may be needed or time for it to remain in place to allow for absorption of blood. In this case, surgeons typically leave it as applied until the conclusion of the surgery since a graft area is not the main target of this surgery. SURGICEL^{®} turns a black color and, as a consequence, makes arteries within the target sites black. This decreases the visibility of the surgical site. Since it has some adhesive properties, SURGICEL^{®} must be removed by tweezers. after application. This step increases time for completion of the procedure and, on occasion, when it is removed, damage to the surrounding tissue may lead to rebleeding. Further, a surgeon is unable to confirm that bleeding is controlled and has stopped from all areas until the SURGICEL^{®} is removed.

Fibrin glue may be applied in the same manner as SURGICEL^{®}, however, as stated above, surgeons prefer using an electrosurgical knife. When fibrin glue is applied in a CABG surgery, it is typically sprayed by attaching a spray nozzle. After removing blood on the target area, it is sprayed by a larger applied pressure or by using compressed air. Fibrin glue requires five to ten minutes to become sticky enough to remain in the location and stop any bleeding. Occasionally, fibrin glue requires pressure using gauze, etc. Fibrin glue does not possess the efficacy to heparinized blood and cannot be applied in advance against bleeding. If it is applied and does not control bleeding sufficiently, it must be removed in order to reapply. This incurs more time on the procedure and the step at which the fibrin glue is being applied. This can also lead to rebleeding. As with the use of SURGICEL^{®}, surgeons cannot confirm that bleeding is controlled and has stopped from all areas until the fibrin glue is removed.

**Bleeding from coronary arteries and after systemic circulation.** After exfoliating the internal thoracic artery, one end is anastomosed to the periphery end of a blocked coronary artery. To accomplish this, the targeted coronary artery needs to be identified from the surface of the heart. However, the heart is covered by adipose tissue and surgeons have to dig into the adipose tissue to find the coronary artery. This can lead to bleeding. If the point of bleeding can be identified, a hemo-clip is applied, and if not, bleeding is widely astricted by gauze, which must be pressed for around a minute and bleeding is controlled. The procedure has to be stopped during this time. Alternatively, peptide compositions described herein can be applied to the area of the coronary artery in advance to prevent bleeding, or when any bleeding is found on the area, since it is transparent and the applied area can be operated by surgical instruments. SURGICEL^{®} and Fibrin glue cannot be applied in advance since they do not accept additional surgical procedures to the area once applied. When SURGICEL^{®} and fibrin glue are applied after bleeding is found, additional time is required due to the need to stop the procedure for application.

**Bleeding from anastomosed arteries.** When the great saphenous vein is anastomosed to a coronary artery, it is performed by thread and needle or by an auto-anastomosing device and bleeding is typically encountered. An electrosurgical knife cannot be used to control this type of bleeding since the burn causes damage to the anastomosed artery. If the bleeding is projectile in nature, additional sutures are made to the appropriate areas. If necessary, fibrin glue, SURGICEL^{®}, or astriction with gauze are applied. SURGICEL^{®} is typically applied to smaller bleeding sites than fibrin glue. In these cases, since the artery has a round shape, application is first conducted to one side of the artery and then, the artery is turned around and application is conducted to the other side. By putting pressure on fibrin glue, the efficacy can be enhanced. However, the mechanism of fibrin glue depends on coagulation of blood itself, which may require more than ten minutes even under pressure.

Peptide compositions described herein are applied easily to the anastomosed arteries. It can be once applied to a finger or gauze then be pasted on the unseen area of anastomosed artery. In this case, instant astriction is possible, unlike fibrin glue, and rebleeding by blood pressure after the application is prevented. This minimizes time under astriction (to 2-5 minutes) and enhances the process of controlling bleeding.

**Bleeding at connecting sites of heart and cannula/tube of an oxygenator.** In CABG surgery when it is necessary to prevent the heart from beating, an oxygenator is connected through a cannula/tube to an artery and heart in order to circulate blood to the rest of the patient's body. The cannula is directly inserted and fixed by sutures. Bleeding is sometimes identified on the suture site during the circulation or on the removal site of cannula after it has been removed. Fibrin glue is typically not applied because it fixes the area and makes removal of cannula difficult. Further, SURGICEL^{®} is difficult to apply due to its sheet-like characteristics. Typically, gauze is pressed and placed on the bleeding point. If the bleeding remains or gest stronger, additional anastomosis is performed. The unique non-glue/non-sheet properties of peptide compositions described herein make it especially applicable to this situation and these types of bleeding encountered during surgery.

**Clinical Study of Composition 1 in human cardiovascular surgery.** Study protocols were approved by the Institutional Review Board of Toho University Medical Center Sakura Hospital and Omori Hospital. Informed consent was obtained from all patients. In this clinical study, 33 application sites in 25 patients (22 men, 3 women) were targeted for application of Composition 1. Patients that satisfied specific criteria and underwent CABG, vascular surgery for abdominal aortic aneurysm (AAA), or arteriosclerosis obliterans (ASO) between January 2010 and April 2011.

The following exclusion criteria was used: (1) individuals with past medical history of hypersensitivity to peptide drugs or protein preparations, (2) individuals with serious complications other than diseases indicated for surgery that may hinder the study, (3) individuals who were unable to discontinue drugs that may affect the use of Composition 1 in the surgical procedure, *e.g.,* blood-clotting drugs (blood coagulation accelerators; *i.e.,* hemocoagulase) and antifibrinolytic agents (*e.g*., drugs with antifibrinolytic action; epsilon aminocaproic acid, tranexamic acid, aprotinin preparations, *etc*.)*,* (4) individuals with child's classification of B or C, and (5) individuals otherwise deemed unsuitable for the study by the investigator.

All procedures were performed while the patient was under general anesthesia. CABG was performed without cardiopulmonary bypass. Heparin sodium was administered during the procedure at 200 IU/kg, and protamine sulfate after the procedure for achieving a target ACT of 200 seconds. Prosthetic vascular graft replacement surgery to treat AAA was performed with a woven Dacron graft (J-graft; Japan Lifeline, Tokyo, Japan). Graft bypass surgery or autologous vein patch plasty to treat ASO was performed with an ePTFE ringed Gore-Tex vascular graft (WL Gore & Associates; Flagstaff, AZ, USA) and saphenous vein, respectively. Heparin sodium was administered during the surgical procedures at 5000 IU, but protamine sulfate was not typically used after the surgical procedure.

In the CABG procedure, target sites designated for application of Composition 1 were vessel-to-vessel anastomotic sites. For surgical procedures to treat AAA and ASO, target sites for application of Composition 1 included the graft anastomotic site and autologous vein patch plasty site. Types of bleeding targeted for application were (1) blood oozing that typically would be arrested with fibrin glue and collagen materials, and (2) blood oozing during typical treatment using other methodology such as ligation, clips, and coagulation that were ineffective or could not be performed. If copious blood spurting or gushing bleeding was encountered, other treatment methodology were typically performed including ligation, clips, or coagulation. Composition 1 was not applied in these situations.

After anastomotic blood was removed with gauze, Composition 1 was evenly applied gently without break down the gelated Composition1 and smeared into each of the target sites before the administration of protamine sulfate. Specifically, approximately 1 mL of 2.5% Composition 1 was applied to coronary anastomotic sites, approximately 2 mL was applied to aortic anastomotic sites, and approximately 1 mL was applied to other peripheral vascular anastomotic sites.

The primary endpoint of Composition 1 that was evaluated was intraoperative bleeding. It was determined as follows: complete response (CR), total arresting of bleeding at the target site; partial response (PR), temporary total arresting of bleeding confirmed, but permanent stoppage of bleeding only observed after reapplying Composition 1 to application sites due to intraoperative secondary bleeding requiring treatment; minor response (MR), temporary stoppage of bleeding confirmed, but permanent stoppage of bleeding only observed after using a procedure other than Composition 1 due to intraoperative secondary bleeding from application sites requiring treatment; no response (NR), bleeding from target sites not reduced and stoppage of bleeding not achieved.

A secondary endpoint of post-operative bleeding was recorded and determined as follows: CR, no post-operative bleeding observed during post-operative examination; PR, post-operative bleeding from Composition 1 application sites inferred from the post-operative examination, without requiring reoperation; and NR, post-operative bleeding originating from Composition 1 application sites observed during the post-operative examination requiring reoperation.

Adverse events including any abnormal findings or adverse reactions were recorded concerning symptoms, severity, duration, treatment, course and outcome, and association with the study drug (as well as the rationale for determining any association).

**Results.** Subjects comprised 25 patients (23 men, 2 women) with an age range of 54-80 years. Of these patients, 9 underwent CABG surgery, 4 underwent AAA surgery, and 12 underwent surgery for ASO. Composition 1 was used on 33 sites, specifically at areas of the internal thoracic artery-coronary artery anastomosis (n = 1), saphenous vein-coronary anastomosis (n = 4), ascending aorta-saphenous vein anastomosis (n = 4), graft anastomosis (n = 15), and autologous vein patch plasty (n = 9). Mean area of the application was 3.03 cm² (ranging from 0.25-10 cm²). Mean amount of Composition 1 applied was 1.5 mL (ranging from 0.5-3 mL). The efficacy rate observed was 87.9% for the primary end-point (intraoperative bleeding) and 100% for the secondary endpoint (occurrence of post-operative after bleeding; Table 4). For heparin treatment, the efficacy rate observed was 85.2% (23/27), and time for stoppage of bleeding was 153.6 ± 38.7 seconds (mean ± S.E.). For the protamine treatment, the efficacy rate was 100% (6/6), and the time for stoppage of bleeding was 195.0 ± 130.1 seconds (mean ± SE). No adverse events (including serious adverse events) having a causal relationship to the application of Composition 1 were observed.

**TABLE 4**

| Application site | No. | 1° Endpoint | 2° Endpoint |
|---|---|---|---|
| Internal thoracic artery-coronary artery anastomosis | 1 | 1 | 1 |
| Saphenous vein-coronary anastomosis | 4 | 3 | 4 |
| Ascending aorta-saphenous vein anastomosis | 4 | 4 | 4 |
| Graft anastomosis | 15 | 14 | 15 |
| Patch suture | 9 | 7 | 9 |
| Total | 33 | 29 (87.9%) | 33 (100%) |

Evaluation of the efficacy of Composition 1 in the clinical study described above was implemented as suggested by Stark et al. (Stark Jet al. 1984, Ann Thorac Surg 38:411-413). Previous have reported total stoppage of bleeding on oozing bleeding at rates of 23.1%-100%. As shown in this example, Composition 1 performs at the top end of this range. Further, what is not assessed in this number is the added benefit of the use of an infection-free material that does not include animal-derived products or human blood components. Because Composition 1 is entirely synthetic, it provides an alternative material that poses no risk of infection.

As shown in this example, Composition 1 was applied to 33 sites in 25 patients and exhibited an efficacy and safety rate of 87.9% (29/33; Table 1). No differences in the efficacy of Composition 1 in heparin- and protamine- treated individuals was observed (data not shown). No post-operative bleeding or other adverse events of any kind were observed. Based on these findings, Composition 1 provides a safe and useful alternative material that demonstrates excellent local stoppage of bleeding on oozing bleeding during cardiovascular surgery.

### Example 4. Thoracic Aorta Replacement

This example illustrates the surgical procedure to replace the region of an aortic aneurysm from the arch to distal region of the aortic aneurysm by total replacement surgery utilizing peptide compositions described herein and, in particular, steps of the surgical method at which peptide compositions described herein can be applied. An exemplary peptide composition provided is Composition 1 (described above).

Briefly, the surgical procedure comprises five steps, (1) aortic cross-clamping and establishment of cardiopulmonary bypass (including cerebral protective reflux), (2) anastomosis of the descending aorta (peripheral side), (3) anastomosis of the ascending aorta (central side), (4) anastomosis of the left subclavian artery, left common carotid artery, and innominate artery, and (5) withdrawal of cardiopulmonary bypass.

**Aortic blockage and establishment of a heart-lung machine (including cerebral protective reflux).** Median sternotomy and pericardiotomy are performed followed by the extirpation of the ascending aorta and heart. Heparin is then administrated. A tube is inserted into the right axillary artery, innominate artery, left common artery, left subclavian artery, and descending aorta in order to reflux blood between a heart-lung machine and the patient. Afterwards, reflux of blood by a heart-lung machine is started during the blockage of each vessel. The heart is stopped by injection of a myocardial protection liquid.

**Descending aortic anastomosis (peripheral side).** The descending aorta is cut by electric scalpel and is anastomosed with a vessel graft with 3-0 or 4-0 Proline thread. Certainty of anastomosis is required as the field view is limited in deep area and it is difficult to stop bleeding after starting reflux.

**Ascending aortic anastomosis (central side).** The ascending aorta is cut by electric scalpel and is anastomosed with a vessel graft with 3-0 or 4-0 Proline thread. The vent tube is inserted into the vessel graft in order to remove air bubbles prior to the restart of blood reflux. The application of fibrin glue on the entire region of anastomosis prior to declamping would decrease the risks of exudative and gushing hemorrhage, however, this procedure may cause bleeding from between the fibrin glue and anastomosed region. In this case, the reapplication of fibrin glue will be required instead of stripping of fibrin glue, since there is a risk of increased bleeding. It is difficult to apply fibrin glue between the anastomosed region and the region previously applied with the glue. Repeated application of SURGICEL^{®} in this instance may not be sufficient to stop bleeding and the final step would be astriction for an extended period of time. long time. In the instance that liquid fibrin glue cannot stop bleeding, sheet type of fibrin glue is employed. If there is further bleeding, fibrin glue is stripped off and then second anastomosis is performed with needle and thread. These steps in the surgical procedure not only increase the volume of bleeding due to the disturbance of anastomosed region where fibrin glue is removed, but also further increases the time of surgery since anastomosis is complicated by the remaining fibrin glue (about 20-90 minutes).

Alternatively, peptide compositions described herein (e.g., Composition 1)can be applied by swab and/or injection on the region where bleeding has not stopped with fibrin glue and the particular site of anastomosis. Application of , e.g., Composition 1 prior to clamping allows for Composition 1 to blend into a shallow layer of blood vessels on the site of anastomosis. A suitable volume of Composition 1 is applied and kept at the site so as not to fall off due to gravity or pushed away from the site due to blood pressure after declamping. During declamping, forceps are slowly removed and about 30 to 60 seconds is elapsed to allow for Composition 1 to gelatinize with blood. The flow of blood vessels from the inferior side is visible, since Composition 1 is a transparent material both in solution and once gelatinized. If Composition 1 is washed or pushed away due to blood pressure, it can be reapplied in repeated fashion until bleeding is stopped while the clamp is retained. Once bleeding is stopped completely, declamping is performed.

**The anastomosis of innominate artery, left common artery, and left subclavian artery.** The innominate artery, left common artery, and left subclavian artery are cut by electrical scalpel and are anastomosed to a vessel graft by 5-0 Proline thread followed by protamine administration. Composition 1 may be used during this procedure as described above.

**The withdrawal from a heart-lung machine (reopened circulation by cardiac beat).**The insertion site of the tube connecting the heat-lung machine and a patient is closed by 6-0 Proline thread. The blood circulation of innominate artery, left common artery, and left subclavian artery is reopened and systemic circulation is resumed.

### Example 5. Lymph Node Dissection

The present Example describes a lymph node dissection utilizing peptide compositions described herein and, in particular, steps of the surgical method at which peptide compositions described herein can be applied. An exemplary peptide composition provided is Composition 1 (described above).

Lymph node dissection of pulmonary hilum and mediastinum is known to be a standard treatment of lung cancer and requires the dissection of the lymph node and surrounding tissue within the anatomical site.

**Left periaortic lymph node dissection.** The mediastinal pleura is incised on the site of left main pulmonary artery into the top of the aortic arch by an electric scalpel. The mediastinal pleura with surrounding tissue is stripped off by using an electric scalpel, scissors and forceps with gauze ball. Lymph node is dissected from the top site of aortic hiatus. In inferior side, while pulmonary artery is exteriorized, the lymph node around the tissue is dissected. The entire lymph node is wrapped with Alice forceps and pulled out. Next, the surrounding vascular and connective tissues are stripped off by electric scalpel, scissors and forceps with a gauze ball. The lymph node is then incised and extirpated by electric scalpel. In cases when the lymph node is adhering to vascular wall or invasively integrating into vascular wall, it remains difficult to follow standard dissection procedure. Thus, the lymph node should be detached under the circumstance that has oozing and gushing hemorrhage, after the astriction for 5-15 minutes and the ligature suture. Otherwise, the lymph node is extracted by clamping blood vessels that would require the reconstruction of blood vessels. In a worst case, the surgery procedures should be converted for the cases such as total extirpation of lung. The conversion of these surgical procedures would f the time of surgery but also increase the risk of postoperative bleeding. Thus, it is ideal to follow standard procedure of dissection.

It is possible to apply peptide compositions described herein (e.g., Composition 1) for the prevention of hemorrhage on the stripped surface. Composition 1 has physical specificity that the gravity slowly pulls it downward, so it is possible to apply Composition 1 not only on the stripped surface but also specifically on the entire lymph node. If desired, a large amount may be used. It is not suitable to use fibrin glue which is solidified. Likewise, SURGICEL^{®}, which covers entire stripped surface in advance of the dissection, is not optimal.

In the case of the dissection without treatment for bleeding beforehand, the dissection can commence immediately after application of Composition 1. If fibrin glue is applied to the region, it is hard to detach and might promote bleeding when stripped off. SURGICEL^{®} cannot be used in this instance since it hides the application site and thus detachment cannot be performed. Further SURGICEL^{®} requires an extend period of time completely stop bleeding.

There is possibility that bleeding is not stopped even after a sufficient period of time and SURGICEL^{®} is removed. This is due to the fact that the stoppage of bleeding cannot be confirmed when SURGICEL^{®} is applied. In this case, it is necessary for reapplication. The bleeding may be mild, however, extensive bleeding can begin again and therefore extend the time of surgery.

### Example 6. Application of peptide compositions described herein in Orthopedic Surgery

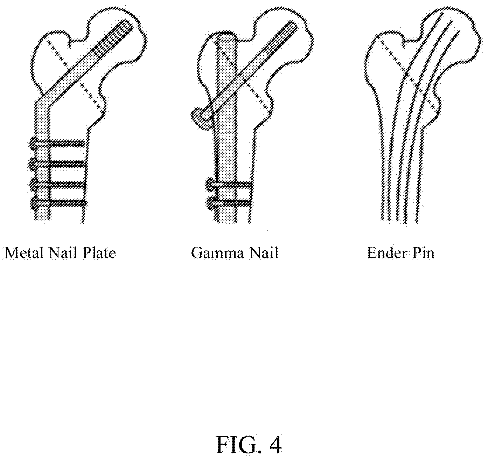

The following example illustrates the application of peptide compositions described herein, e.g., Composition 1, in a surgery to repair an intertrochanteric hip fracture (Figure 4). The site of fracture is fixed by metal nail plate or gamma nail that can bear 3 to 5 times of weight. In recent years, with modernization of surgical techniques and metal fixation devices, the compression forces on the fracture site are made by screws and plates (or triangular nail) that are introduced into fractured bone to prevent the fracture site on the plane from sliding.

**Gamma Nail for intertrochanteric fracture with application of Composition 1.** First, a skin incision is made at the fracture site. A small incision is made deep through the fascia lata, splitting the abductor muscle to reach the targeted femur. When encountering bleeding during incision, normally, astriction by gauze is performed. Five to ten minutes is necessary for to stop the bleeding and the procedure should be stagnant. Also, control of bleeding via additional coagulation by high frequency wave electro device needs substantial procedure time to apply frequent times. Alternatively, Composition 1 presents an advantage for faster control of bleeding by application over a wide area of bleeding, which may include multiple bleeding points. Further, due to the transparent nature of Composition 1, there is no obstacle or impairment in the surgical field and thus the procedure can proceed as normal without any delay.

After exposing the femur by incision of muscle, a guide wire is introduced into the canal of femur from the top of the great trochanter before the introduction of a gamma nail into the appropriate position. Then, a hole is made by a reamer along with the guide wire with a suitable diameter for introduction of the nail. At this point in the procedure, bone wax is typically used for controlling any bleeding from the femoral canal. Bone wax is a clay-like material that requires kneading and/or warming prior to use. Typically this can be accomplished manually by the surgeon's fingers. Alternatively, Composition 1 can be used instead of bone wax. Preparation before application such as for bone wax is not necessary for Composition 1 and faster control of bleeding is achieved. Specifically, Composition 1 can be applied by a pre-filled syringe with a nozzle adapted for the hole in the bone or fracture depending upon the type of fracture or repair made to the bone. Further, Composition 1 provides the added benefit of not stopping the procedure for its transparent quality in maintaining a clear surgical field and easy removal by irrigation.

Although the applications of both bone wax and Composition 1 are similar regarding their application on a given bone or bone fracture site, bone wax tends to delay bone synostosis, whereas Composition 1 promotes bone synostosis and is expected to have higher efficacy of healing than bone wax. Further, bone wax can cause inflammation whereas Composition 1 does not, due, in part, to its high biocompatibility.

A gamma nail is introduced into the femoral canal opened by the reamer. Bone wax is used when bleeding from the canal during this procedure. Alternatively, Composition 1 is applied at this point and no stoppage in the surgical procedure is incurred. Further, control of bleeding is achieved application of Composition 1 onto the surface of the gamma nail before introduction.

Before the introduction of a lag screw into the femoral head, the entry point at the lateral femur is determined by a dedicated instrument. Then, a skin incision is made at the entry point through the fascia lata, splitting the abductor muscle to reach the targeted bone. When bleeding occurs, typically astriction by gauze or coagulation is performed onto the bleeding sites. Alternatively, Composition 1 is applied to achieve faster control and stoppage of bleeding. This application is suitable for a pre-filled syringe which can be used for targeted application. This decreases overall time to complete the surgical procedure which leads to a faster recovery for patients.

The lag screw is selected by considering the size of the bone and position of the fracture site. Then an appropriate diameter and length for the hole to accept the lag screw is determined and the hole for lag screw is made by reaming from the lateral side of the femur toward just below the center of the femoral head. Next, a lag screw is inserted into this hole and the nail and the lag screw is fixed. This results in fixation between fracture of the intertrochanteric part and core of femoral bone. When bleeding results from the hole of the lag screw after reaming the bone, typically bone wax is placed into the hole manually to control the bleeding. Alternatively, Composition 1 can be placed or injected in the same manner as the bone wax which will provide more effective bone synostosis.

In order to fix the introduced nail with femur, screws are inserted into the holes of the nail and the core of femur vertically. Skin incisions are made at entry points through the fascia lata. The abductor muscle is slit to expose the targeted femoral bone and screws are inserted into the nail after making holes at the femur by reamer. During this procedure, astriction by gauze or coagulation is performed against bleeding from the skin, abductor muscle and the bone marrow like the procedure with introducing the nail into the femoral canal. Alternatively, Composition 1 can replace the astriction by gauze and can be applied in a targeted manner, if desired, by using, e.g., a pre-filled syringe with an special nozzle to control the flow onto the desired location. Standard techniques employ methods for controlling bleeding separately depending on site (e.g., skin, muscle and bone). Conversely, Composition 1 can be applied to different sites regardless of tissue with a single procedure or methodology, which can eliminate complicated procedures and decrease the overall time required for surgery.

Surgery is completed by closing the abductor muscle, fascia lata and skin by suturing. In this procedure, astriction by gauze or a number of sutures are increased when bleeding occurs from the incision sites. Alternatively, Composition 1 can be used effectively at this point in the procedure by direct application on the incision site by a syringe, which can shorten the procedure time and reduces the number of sutures required. The surgical field at which the sutures are being made is not hindered due to the transparent nature of Composition 1. This application will not add any additional time to the procedure and can expedite closing the surgical site appropriately.

### Example 7. Surgical Resection of the Liver (Hepatectomy)

The present Example describes surgical resection of a liver utilizing peptide compositions described herein and, in particular, steps of the surgical method at which peptide compositions described herein can be applied. An exemplary peptide composition provided is Composition 1 (described above).

Hepatectomy is typically performed using an ultrasonic surgery suction unit, an ultrasonic solidification incision equipment and an electric scalpel. The blood circulatory system and funicular objects in the liver are exposed by an ultrasonic surgery suction unit. The hemorrhage from a bile duct portal vein or a thick vein are normally litigated, and from funicular objects thinner than 3-0 thread are arrested by a ultrasonic solidification incision equipment. Ischemia-reperfusion of the liver is also performed to reduce the amount of bleeding by repeating the clamp and release of the blood circulatory system of the liver.

It remains difficult to use an electric scalpel near the vascular of the liver, especially around Glisson's Capsule, due to the risk of vascular injury. Exudative bleeding, which comes out gradually from the part cauterized by an electric scalpel cannot be stopped. Furthermore, re-bleeding may occur by stripping the scab when an electric scalpel is applied near to the part where bleeding has already stopped. An electric scalpel can be used, however, on the part to which peptide compositions (as described herein, e.g., Composition 1)have been applied. The drawbacks in using SURGICEL^{®} and fibrin glue is that additional liver separation or vascular exfoliation cannot be performed on the site of application. Moreover, it is difficult to remove the fibrin glue without causing damage to the peripheral tissues. However, Composition 1, for example, provides the opportunity for additional treatments to be performed onto the site of application, and little to no damage is caused to the vascular system. Thus, total surgery time is decreased.

The use of pressure to arrest a hemorrhage is typically carried out with gauze when a bleeding point is not clearly identified. SURGICEL^{®} is also applied, however, the efficacy is low because coagulability of a liver separation in a patient is typically low. Fibrin glue is not applied in this instance, since fibrin glue is solidified to the tissues other than the bleeding point, and it is difficult to remove. Composition 1, for example, can be applied without hesitation in this instance, because the hemorrhage efficacy is high and it is easily removed by suction, gauze or washing. In the instance of endoscopic or laparoscopic surgeries, identification of the bleeding point is much more difficult. This indicates a more advantageous situation for application of peptide compositions, e.g., Composition 1.

SURGICEL^{®} can be applied to oozing if a bleeding point is clearly identified, however, it must be removed, unlike peptide compositions, before carrying out additional treatments. This increases the time required to control bleeding significantly. Peptide compositions described herein are superior since additional treatments can be performed onto the application site and the operation is not interrupted by to process of stopping bleeding. Fibrin glue cannot be used during liver separation due to the difficulty of removing it after the stoppage of bleeding.

Peptide compositions described herein may be applied repeatedly until complete stoppage of bleeding is achieved. In the case of liver separation, it is preferable to apply Composition 1, more than 1mL to one bleeding site. On wet surfaces, Composition 1 may not remain stationary and collapse if manually manipulated, e.g., rubbed with fingers. Composition 1 is be applied to a larger area than the bleeding site itself. Surplus of Composition 1 is neglected during liver separation because it is easily washed out after completion of the surgical procedure.

When complete stoppage of bleeding is not achieved after an application of Composition 1, additional application can be performed toward the bleeding which comes out from the applied Composition 1. Exudative bleeding from a large area can be also effectively stopped by the above method. In the case fibrin glue, surgeons must remove and re-apply the fibrin glue or excessively apply the fibrin glue to the surroundings of the solidified fibrin. Surgeons may avoid the excessive application altogether, since application of fibrin glue to the surrounding areas does not stop the bleeding point directly. Sometimes bleeding comes out from under the fibrin glue. Composition 1 can be injected into the gap to achieve more effective stoppage of bleeding during the surgical procedure. Fibrin glue is usually sprayed onto the section to stop minor bleeding and prevent post-hemorrhage at the end of the liver separation procedure. Composition 1 is suited for this step in the surgical procedure and can be applied onto the section after liver separation is completed. Typically, SURGICEL^{®} is not used effectively in liver separation due to the effect of peeling off the section.

### Example 8. Pure Laparoscopic Hepatectomy (PLH)

The present Example describes a laparoscopic surgical procedure of a liver utilizing peptide compositions described herein and, in particular, steps of the surgical method at which peptide compositions described herein can be applied. An exemplary peptide composition provided is Composition 1 (described above).

Pure Laparoscopic Hepatectomy (PLH) is typically performed in situations where pathological lesions are on the surface of the liver, however, it can also be performed in situations of partial hepatectomy and liver lobectomy.

Briefly, a camera-port is inserted into the umbilical region and 2-3 ports are inserted near the tumor after the tumor location is confirmed. Then, 3-4 ports are employed for the surgical procedure. The relationship of the tumor and the vascular system is typically identified by ultrasonography. This is due to the fact that direct contact to the tumor is not made in a PLH procedure. Next, the resection line is determined and marked by electric scalpel. In an effort to reduce bleeding during the resection, pre-coagulation is performed (e.g., microwave coagulation and radiofrequency ablation).

The resection of the shallow layer is performed by an ultrasonically activated scalpel. The large vascular in the deep zone of the liver is exposed by the ultrasonic surgical aspirator that is used in the rupture and suction of hepatic parenchyma. The oozing-type bleeding that that can be clearly identified during the hepatectomy is cauterized by utilizing an electrosurgical knife. Any additional bleeding is stopped by ligation. If any oozing-type bleeding occurs during hepatectomy without the identification of the apparent bleeding points, considerable time would be added to the surgery for the following the reasons: the visual field is limited in laparoscopic surgical procedure as compared with open laparotomy surgical procedure, the bleeding is coagulated by ultrasonically activated scalpel while blood is removed by gauze to ensure the filed view, SURGICEL^{®} is applied to the bleeding area when coagulation is not performed, which can incur extensive additional time because (1) difficulty in swabbing on the targeted region, especially the back side of the transection in laparoscopic surgery as SURGICEL^{®} is made from cotton and (2) the bleeding spreads while forceps are clamping SURGICEL^{®} in place and going in and out of one of the ports, and the blood is removed by gauze or washed away by saline solution to ensure the surgical field can be viewed clearly in order to apply SURGICEL^{®}.

Peptide compositions, for example Composition 1, can be applied to the back side and wide range of the bleeding area since it can quickly spread out in one application through a tube. This is a unique advantage of Composition 1 as it is applied as a liquid and adopts a gelled state once on the tissue or surgical site. Conversely, fibrin glue is not suitable during the resection because of the hardened effect it has on the section and further makes it difficult for the surgeon to perform the resection.

The bleeding points should be identified thoroughly in the sections of the vascular and hepatic parenchyma after hepatectomy. Typically, the exudative bleeding is stopped by coagulation using an ultrasonically activated scalpel and by swabbing with fibrin glue. The coagulation delays the hepatic regeneration due to the carbonization of the tissue. It is difficult for the fibrin glue to remain on the sections and tends to flow downward. Further, since the fibrin glue solidifies and persists at the surgical site, an enhanced risk of infection at the site of surgery occurs. Alternatively, application of Composition 1 reduces such risk due to, at least, Composition 1 is easily washed away due to the gelled state that results after application to the bleeding sites.

### Example 9. Thoracoscopic Partial Lung Resection

The present Example describes intrabody surgical procedure of a lung utilizing peptide compositions described herein and, in particular, steps of the surgical method at which peptide compositions described herein can be applied. An exemplary peptide composition provided is Composition 1 (described above).

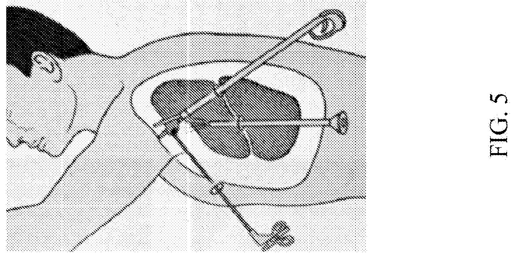

In the first step of this procedure, a surgeon first identifies an excision site and inserts a trocar and thoracoscope into the chest between ribs through a small incision (Figure 5). The surgeon then checks the area to be resected through a thoracoscope and sets the direction for autosuture. Next, an autosuture is inserted into the chest through a small incision. Typically, an area about 1.5 to 2 cm away from the resected area is gripped by forceps in order to indicate the line to be resected by autosuture. Lung tissue needs to be gripped carefully because it can be easily torn off if gripped too tightly and pulled in a strong manner. If the tissue was not cut straight by autosuture, the resected area is reinforced by suture, absorbent mesh or collagen sheet. For suturing, a suture thread of 2.0 or thicker is generally used. For ligation, tying is conducted outside the body and ligature is sent using a knot pusher inside the chest.

If lung tissue or vessel is unintentionally cut during resection or damaged by a suture needle, gauze astriction is first used to stop bleeding. If bleeding is not stopped by gauze astriction, fibrin glue, is applied to the bleeding site. When liquid form fibrin glue is applied, surgical field may be blocked because fibrin glue is not colorless or transparent. Fibrin glue and poly-glycolic acid (PGA) sheets are used concurrently as a standard method to control and stop bleeding, although the use of these materials takes added time and can be burdensome for the surgeon. Under this procedure, a PGA sheet is first attached to the bleeding site, and then fibrin glue is applied onto the area. Subsequently, a surgeon is required to wait for at least five minutes and check the status of the bleeding.

When a pulmonary artery is damaged during the surgery, the surgical field is almost lost entirely due to extensive bleeding. As such, the bleeding site can be difficult to identify. Unless the bleeding site is clear, fibrin glue tends not to be used at this point so as to avoid solidification of tissue around the bleeding site caused by the application of fibrin glue. To stop the bleeding, a surgeon routinely attempts to roughly identify the bleeding site and performs astriction or blocks the vessel. If these attempts do not work, the chest is opened for additional measures.

At the end of the surgical procedure, a thoracotomy tube is inserted to check the status of bleeding. If bleeding is detected, a draining procedure is first taken to remove blood remaining inside the chest. Then, the other measures (as described above) are also performed. If fibrin glue was applied for the first attempt to stop the bleeding, a second application cannot be performed easily because tissue can be torn and additional bleeding sites can occur or the original bleeding site can expand when it is removed. When bleeding is not recovered under thoracoscopy with draining and the other methods described above, the chest is opened for additional measures.

After surgery, a sealing test is also performed to reveal air leaks. This test is conducted by using an airway pressure of 5 to 10 cm H₂O. Any major air leakage is stopped with suturing. Main adjuncts to prevent air leak are bovine pericardium, Gore-Tex or autologous pleura. Although buttressing of the staple line has been shown to reduce the duration of an air leak, associated staples of buttressing sometimes results in tissue trauma.

If any lung tissue or vessels were damaged during the resection or by suture needle under thoracoscopy, surgical field can be blocked with only small amount of blood. Application of peptide compositions described herein, e.g., Composition 1, stops bleeding without blocking the surgical field due to its transparent qualities, which is direct contrast to fibrin glue. Because Composition 1 is administered in liquid form, it can also be directly injected, e.g., by syringe, into the bleeding site easily through the tube and applied well onto the surface of lung unlike any sheet-type material. Further, application of Composition 1 does not require astriction nor does it hinder the surgical procedure. Composition 1 can also be left after being applied and surgeon can check the status of bleeding at any time.

If the pulmonary artery is damaged during the surgery, treatment for bleeding is required immediately. However, unlike open chest surgery, it normally takes time to identify bleeding site and is difficult to conduct astriction. Composition 1 shows sufficient control and stoppage of bleeding for a lung artery with low pressure. Again, because Composition 1 is transparent, excess can be removed easily by draining after treatment. Any volume can be applied to the area around the bleeding site without hindering the surgical field at any time, thereby allowing the surgeon to resume the surgical procedure swiftly.

If bleeding is detected again at the end of surgery, unlike fibrin glue, Composition 1 can be removed easily from the bleeding site and applied any number of times to the same site.

For repairing any air leak, Composition 1 can be applied to the site of leaking air easily through the tube and applied well to the surface of lung unlike sheet type products. Application of Composition 1 can shorten duration of surgical operation compared to buttressing. Unlike buttressing, application of Composition 1 does not harm lung tissue by needle. If air leakage is detected again after leakage site is treated, Composition 1 can be removed easily and applied any number of times to the same site quickly.

### Example 10. Endoscopic Mucosal Resection (EMR)

The present Example describes endoscopic surgical procedure of the gastrointestinal system utilizing peptide compositions described herein and, in particular, steps of the surgical method at which peptide compositions described herein can be applied. An exemplary peptide composition provided is Composition 1 (described above).

Generally, endoscopic mucosal resection (EMR) is accepted as a treatment option for cases of early gastric cancer where the probability of lymph node metastasis is low. EMR is applied to patients with early cancers up to 25 mm in diameter that are of a well or moderately histologically differentiated type, and are superficially elevated and/or depressed but without ulceration or definite signs of sub mucosal invasion. Most EMRs are performed by a "strip biopsy method", a relatively simple technique that has been described elsewhere. A more recent EMR procedure has been developed the employs an insulation tipped diathermic knife (IT knife), which is used in the majority of cases (as described below). The IT knife consists of a conventional diathermic needle knife (KD-1L; Olympus, Japan) with a ceramic ball at the top to minimize the risk of perforation. Follow up endoscopy is performed at three and six months post EMR.

Typically, an EMR procedure is conducted as follows: (a) superficial elevated early gastric cancer is identified on the lesser curvature of the lower body after spraying with indigo carmine dye, (b) marking dots are made using a precut knife on the circumference of the target lesion to clarify the margin, (c) after injection of saline or hyaluronic acid with epinephrine (0.025 mg/mL) into the sub mucosal layer, an initial cut is made with a conventional needle knife outside of the dots and an IT knife is inserted into this cut and employed to cut around the lesion, (d) the marked tumor is separated from the surrounding normal mucosa, (e) the tumor is removed by standard polypectomy with a combination of cutting and coagulation current in a single fragment, and (f) the resected specimen shows well differentiated adenocarcinoma (20 x 25 mm) with a clear lateral margin.

The control of bleeding is very important in this procedure. If the bleeding is very severe, blood transfusion or surgery will be considered. When bleeding occurs, especially during resection by an IT knife and polypectomy, normally endoscopic treatment with coagulation, ethanol injection, endoscopic clipping, spraying of thrombin solution or combination of these treatments are used as the situation demands. However, these treatments have disadvantages. Regarding coagulation, damages to the surface of the tissue can cause bleeding because the technique employs using a loop wire to perform the polypectomy and resection at the bleeding site. In this case the tissue has no time to heal. Regarding ethanol injection, a low efficacy for controlling bleeding and risk of enlargement of an ulcerated area can result if too much is injected onto the bleeding site via a syringe through the endoscope. Regarding clipping, sufficient time and skill are required of the surgeon, and with more time spent on the surgical procedure more unseen errors can occur, e.g., rupture of muscle walls. Regarding spraying a thrombin solution, low efficacy for controlling bleeding is seen especially for exposed vessels with an obscured vision of the treatment field due to its opacity. This is due to it being sprayed at the bleeding site through a catheter through an endoscope.

Peptide compositions, such as, Composition 1 is applied first through a catheter after the initial cut around the lesion is made by the IT knife. This is due to prevention of bleeding at the time of resection by polypectomy. This is suitable for Composition 1 as it is a solution and gels upon contact with the bleeding site. This application does not hinder the time of the surgery and the surgeon can quickly proceed due, in part, to clear treatment field created by application of Composition 1. Further, due to its ability to adopt a gelled state once applied, it remains around the lesion site to prevent bleeding. Thus, application of Composition 1 before resection may largely reduce the risk of bleeding and this may enable the elimination of the use of coagulation during resection.

If bleeding occurs during resection by, e.g., a deeper incision than anticipated, Composition 1 is applied liberally to the bleeding site after irrigation. This is not only to stop bleeding but to keep the surgical field clear in order to identify the bleeding site. The application of Composition 1 eliminates the need for conventional methods such as ethanol injection, endoscopic clipping or spraying of thrombin solution. Control of bleeding during surgical procedures by these methods needs substantial procedure time to apply to multiple points. Conversely, application of Composition 1 in surgical procedures described herein provides better stoppage and control of bleeding over a wide area that includes multiple bleeding sites.

However, if the bleeding is severe such as oozing, spouting, gushing or exposed vessels, normally clipping or coagulation is used. Composition 1 can supplement these methods in an efficient manner to prevent further bleeding and decrease times spent by surgeons addressing such situations.

Composition 1 is applied just after removal of tumor to prevent post operating bleeding at the site of resection. This procedure eliminates the need for clipping and shortens the time of procedure for about 10 minutes.

As shown in this example, application of Composition 1 eliminates the need for combination of the procedures described above for controlling bleeding during tumor resection before, during and after the resection as mentioned above and can shorten the total time of procedure. It is projected that tumor resection can be decrease on average of at least 20 minutes. Also, patient safety is increased as compared to the other techniques, due to the decreased bleeding and damage to surrounding tissues throughout the procedure. Thus, faster patient recovery is expected.

### Example 11. Endoscopic Sub mucosal Dissection (EDS) For Colon

The present Example describes an endoscopic surgical procedure of a colon utilizing peptide compositions described herein and, in particular, steps of the surgical method at which peptide compositions described herein can be applied. An exemplary peptide composition provided is Composition 1 (described above).

Generally, ESD is applied to patients with early cancers larger than 20 mm in diameter that are hardly resected by EMRs. A team of a surgeon and an assistant perform the procedure using a colonoscope (PCF-Q260AI; Olympus, Tokyo, Japan) in addition to other surgical instruments. The typical procedure for ESD is as follows:

Indigo carmine dye is sprayed to identify the lesion margins of the colon, followed by an injection into the sub mucosa to lift the lesion. A mixture of 10% glycerin and hyaluronic acid containing 0.5% indigo carmine and 0.1% epinephrine is used as the injection fluid. Next, a circumferential incision is performed using an instrument such as needle knife, insulated-tip knife (KD-610L, 611L; Olympus, Tokyo, Japan), or flush knife (DK2618JN20; Fujinon, Tokyo, Japan) that is connected to an electro surgical unit, according to the surgeons preference. Continuous sub mucosal dissection along the circumference of the target lesion is performed using one of the above mentioned instruments. Bleeding is controlled by specialized forceps or an insulated-tip knife during the procedure. Once ESD is completed, coagulation of visible vessels in the dissection area is also performed using specialized forceps or an insulated-tip knife to prevent delayed bleeding. Despite this measure, post-ESD bleeding cannot typically be controlled in an efficient manner. If vomiting or discharge of blood occurs after operation, emergency endoscopic efforts to control bleeding are performed using specialized forceps or an or insulated-tip. Typically, post-operative bleeding is relatively minor, however, bleeding by tissue necrosis caused by excessive coagulation is often encountered during the surgical procedure.

Alternatively, peptide compositions described herein, such as Composition 1, are applied first on the circumference of the target lesion through a catheter after the marginal incision is made. In this case, Composition 1 is poured or injected (e.g., by a syringe) into the inner tissue from the circumference of the incision. This is to prevent bleeding at the time of dissection typically encountered by usage of an insulated-tip knife or flush knife. This application of Composition 1 establishes a clear surgical field due to its transparent qualities and allows the surgeon to proceed quicker to the next step of the procedure. Further, due to the gelled state that results from contact with the tissues and fluids, it remains on the circumference of the incision thereby preventing bleeding from occurring. Thus, Composition 1 application before dissection can reduce the risk of bleeding. As a result, this further reduces the frequency of using specialized forceps for coagulation during dissection.

If bleeding occurs during dissection by, for example, a deeper incision than anticipated, Composition 1 is applied liberally at the bleeding site after irrigation of blood. This not only stops the bleeding but also keeps the surgical field clear in order to identify the point of bleeding. The application of Composition 1 eliminates the coagulation treatment by specialized forceps or an insulated-tip knife. Stoppage of bleeding during the surgical procedure by such instruments requires substantial procedure time to apply to multiple points. Conversely, control of bleeding by Composition 1 provides a better alternative by controlling bleeding in a more efficient manner by enabling application over a wide area that includes multiple bleeding points and simultaneously maintains a clear surgical field allowing the surgeon to more efficiently complete the surgical procedure.

In cases where the bleeding is more severe than oozing, such as spouting, gushing, typically coagulation by specialized forceps or insulated-tip knife is applied. When coagulation is done by an instrument, Composition 1 can be applied onto the operating field to prevent further bleeding. Unlike the treatment by these instruments, Composition 1 does not render any damage to the tissue surface whereas specialized forceps or an insulated-tip knife tend to lead to tissue necrosis and complicates patient recovery. Further, extensive coagulation yields severe tissue necrosis that leads to further delayed bleeding. The advantages of Composition 1 in this procedure are as follows: faster stoppage of bleeding just after application that shortens the procedure time by at least about five to ten minutes, a clear surgical field that enables the visualization of the point of bleeding due to its transparent quality, renders no damage to tissue surface by application.

Composition 1 is applied just after completion of ESD to prevent post-operative bleeding at the site of dissection. This procedure eliminates the use of specialized forceps. This can further shorten this part of the surgical procedure time by at least about five to ten minutes. Composition 1 is applied liberally on the lesion site if bleeding by vomiting or discharge occurs after the procedure. This application eliminates the need for coagulation by specialized forceps or an insulated-tip knife. This again leads to a shorten procedure time and benefit to the patient.

As shown in this Example, the application of Composition 1 can largely reduce the frequency of coagulation by instruments during and after dissection and will shorten the time of the surgical procedure on average by at least about 20 minutes (this may vary based on situation present by each patient). Further, as compared to the coagulation techniques, patient recovery is observed to be faster due to the less projected bleeding and no tissue necrosis during the procedure and preserving tissue on the surface of the dissected tissue.

### Example 12. Open Partial Nephrectomy

The present Example describes an intrabody surgical procedure of a kidney utilizing peptide compositions described herein and, in particular, steps of the surgical method at which peptide compositions described herein can be applied. An exemplary peptide composition provided is Composition 1 (described above).

The following example describes the steps of an open partial nephrectomy. Briefly, a patient is laid at half lateral position and skin incision is performed (celiotomy). The retroperitoneum is then stripped and extended, the lateroconal fascia is exposed and incised by using an electrical scalpel. Gauze astriction is applied when oozing or gushing bleeding is encountered. The renal artery, renal vein and urinary duct must be identified before proceeding further.

Gerota's fascia, a smooth capsule membrane of kidney is then decapsulated by using a harmonic scalpel. Since Gerota's fascia consists of numerous capillary vessels, decapsulation is performed in a careful manner and small oozing bleeding is typically cauterized. Gauze astriction is also applied to any massive bleeding. Complete decapsulation is sometimes performed for identifying the tumor location. Connective tissue between peritoneum and anterior surface of the kidney is also stripped thoroughly. In case of a subsequent follow-up surgery, fusion fascia must be stripped, which most often invokes oozing bleeding and cauterization is required to stop such bleeding. This adds considerable time to the surgical procedure. The location of the tumor is identified by using an ultrasonic probe. Next, arterial clamping is performed and should be released within 30 minutes. Connective tissues around the renal artery and urinary duct are stripped. Typically, gauze astriction or SURGICEL^{®} is applied to minimize the bleeding (oozing or gushing) from the stripping area. In instances where the perinephria is very hard, complete stripping of the connective tissue is difficult because forced stripping of the connective tissue results in rupture of the tumor capsule. However, it does inhibit the detection of the tumor location.

The kidney is cooled with ice for about five minutes to avoid ischemia-reperfusion injury. On occasion, a mannitol solution is applied onto the kidney surface, and blood flow of renal artery is shut off using clamping forceps. In some cases when the distance between the renal calyx and the tumor or between the renal sinus and the tumor is far enough (farther than 1 cm), the clamping of the renal artery is not performed. However, this can cause a larger amount of bleeding. Normal renal tissue of 0.5 to 1 cm outside the tumor location is dissected with coagulotomy by harmonic scalpel or by Metzenbaum. Gauze astriction is applied to the dissected surface and oozing or gushing bleeding is stopped by using an electrical scalpel, argon beam coagulator, fibrin glue or ligation. Control of bleeding is important at this point in the procedure, so continuous astriction by finger is often performed. In case the renal calyx is opened, ligation and Z-suture is performed to close the renal calyx. If major bleeding is not identified, continuous suturing with a suturing clip or only renal parenchyma suturing is performed instead of ligation. Then leakage of urine is checked by applying indigo carmine solution.

Blood flow is reperfused within 30 minutes by releasing the clamping forceps. Stoppage of bleeding is confirmed at this time. If identified, additional cauterization, SURGICEL^{®}, fibrin glue or thrombin is applied. Sometimes bleeding site is covered by adipose tissue or SURGICEL^{®} and is sutured as a whole (mattress suture).

Since Gerota's fascia consists of numerous capillary vessels, blood oozing is likely to be occurred (as described above). Peptide compositions described herein, such as Composition 1, can be applied liberally in advance or just after bleeding occurs to the large blood vessels, which are not desired to be coagulated or ultrasonically coagulated. This application retains a clear surgical field due to the transparent quality of Composition 1 and remains washable.

It has been reported that complete stripping of the surrounding perinephria tissue is important for identification of the tumor location. Liberal or conservative application of Composition 1 enables efficient control of bleeding from the perinephria, thereby allowing for complete stripping. The fusion fascia is stripped by electric scalpel with applying tension by hand. The risk of oozing or major bleeding is increased by this procedure. Control of bleeding can be achieved by applying Composition 1 in advance or just after bleeding occurs as desired. This does not affect the following procedures due to the transparent and washable qualities of Composition 1. The application of Composition 1 is not restricted. Fibrin glue or other bleeding control measures are not typically applied at the step of stripping the perinephria as an ultrasonic probe is typically used.

Composition 1 can be applied to the connecting tissue of renal artery unlike fibrin glue that may cause damage to the artery when removing it. SURGICEL^{®} can be applied to the renal artery, however, it must be removed before proceeding to the next step in the procedure. Alternatively, Composition 1 can be removed by washing with saline. Thorough stripping of connective tissue is achieved by applying a liberal amount of Composition 1 in advance. This enables the exact detection of the tumor location. If the tumor location is obscure, a larger margin must be dissected for secure extirpation of the tumor. The application of Composition 1 does not prevent identification of the tumor location due to its transparent and washable qualities.

In case the renal artery clamping is not performed, considerable oozing bleeding often occurs. Application of Composition 1 in advance prevents such bleeding, and, concomitantly ensures a clear surgical field. Complete control and stoppage of bleeding can be confirmed by removing an excess amount of Composition 1 by washing with saline before closing the abdominal cavity.

The limitation of ischemic time of 30 minutes will be a primary reason to prevent the detection of the tumor location because it is difficult to achieve complete stripping of connecting tissue within 30 minutes. Since application of Composition 1 reduces the surgical time spent controlling bleeding and avoidance of artery clamping, it provides more secure surgical environment during partial nephrectomy.

The milder dissection method can be chosen by bleeding control by the prior application of Composition 1. However, even if it applies, use of a harmonic scalpel is not restricted. Application of Composition 1 in advance or instantly to an oozing bleeding site will attain prevention of bleeding and a clear field of view. Operation time is saved by substituting application of Composition 1 for gauze astriction. The pressure arrest of bleeding with fingers is continued in many cases, and when sufficient arrest of bleeding is not obtained, fibrin sheet and fibrin glue may be used. Composition 1 can be substituted over the above procedures, and since the surgeon can shift to other procedure of operation, the operation time will be significantly saved.

There is also an opportunity to apply Composition 1 to the circumference after the suture of the renal calyx. This contributes to the prevention of post-bleeding. Since Composition 1 is washable, it does not prevent checking for a urine leak. A minute leak hole can be prevented by Composition 1, and perhaps only by Composition 1, which makes ligation unnecessary and further reduces time performing the surgical procedure.

Less than 30 minutes of ischemic time is desired. In a temporary arrest of bleeding, fibrin glue and SURGICEL^{®} lead to an obstructed view of the surgical field and thus an excessive amount of time is added as it is necessary to remove these materials. Composition 1 has an advantage of being able to advance the procedure without flushing the surgical field to maintain a clear view point.

Blood flow can be reperfused for a while in the situation where bleeding remains by applying Composition 1 to the dissection area. Additional bleeding control measures may be performed under blood flow. The application of fibrin glue here is restricted as it must be peeled off to remove, while Composition 1 can be removed simply by washing. Although the method of mattress suture is also taken, Composition 1 can be substituted as well. Composition 1 has the potential to operate as a prevention of post-surgical bleeding control without the need for washing after application. The postoperative complications associate with open partial nephrectomy include urine leak (0-9%) and postoperative bleeding (1-9%) and the requirement for a positive arrest of bleeding is high.

### Example 13. Laparoscopic Partial Nephrectomy

The following example illustrates a laparoscopic surgical procedure for partial nephrectomy, some steps of which are described above in Example 12, utilizing peptide compositions described herein and, in particular, steps of the surgical method at which peptide compositions described herein can be applied. An exemplary peptide composition provided is Composition 1 (described above).

Briefly, a patient is laid at half lateral position and holes (at least four) for torocar are made. Because of the need to cut off blood flow, a flexible port for clamping forceps is typically prepared. Next, the retroperitoneum is stripped and extended, and the lateroconal fascia is exposed and incised by using an electrical scalpel while controlling any bleeding. The locations of the renal artery, renal vein and urinary duct must be identified in order to proceed.

Gerota's fascia, which is a smooth capsule membrane of kidney, is decapsulated by using a harmonic scalpel. Since Gerota's fascia consists of numerous capillary vessels, decapsulation is performed in a careful manner so as not to trigger bleeding. Complete decapsulation is sometimes preferred for identifying the tumor location. Connective tissue between the peritoneum and anterior surface of the kidney is also stripped thoroughly. The location of the tumor is identified by using an ultrasonic probe.

Arterial clamping should be released within 30 minutes. Connective tissues around the renal artery and urinary duct are stripped. Typically, SURGICEL^{®} is applied to minimize bleeding (oozing or gushing) from the stripping area. After stripping, the kidney is cooled with ice for about five minutes, and blood flow of the renal artery is shut off by clamping with forceps. Because the operative field often becomes less visible due to bleeding from the transected renal surface, the renal vein is also stripped and blood flow of renal vein is often shut off in laparoscopic partial nephrectomy.

Normal renal tissue of 0.5 to 1 cm outside of the tumor location is dissected with coagulotomy by a harmonic scalpel. To prevent major bleeding, dissection is performed while simultaneously controlling any bleeding. While lifting the tumor, coagulotomy of the root mass is performed. If bleeding occurs, an electric scalpel, fibrin glue or ligation is employed to arrest the bleeding. If the renal calyx is opened, ligation and Z-suture is performed to close the renal calyx followed by confirmation of no leakage of urine by application of indigo carmine solution.

Blood flow is reperfused within 30 minutes by releasing the clamping forceps. Confirmation of no bleeding is made. If bleeding is identified, additional procedures such as cauterization, SURGICEL^{®}, fibrin glue or thrombin is employed.

Since Gerota's fascia consists of numerous capillary vessels, oozing-type bleeding is likely to be encountered. By the applying peptide compositions describe herein in advance, bleeding can be controlled and stripping can be proceed without disturbing the surgical field. Because an ultrasonic probe is used to identify the location of the tumor, conventional measures for arresting bleeding, such as fibrin glue, is difficult as identification of the bleeding site is necessary prior to employing such measures. Peptide compositions described herein can be washed away by washing with saline, so it can be used for arresting bleeding encountered during stripping.

For example, Composition 1 can be applied to the connecting tissue of the renal artery unlike fibrin glue. This is because fibrin glue can lead to damage to the artery when removing it. SURGICEL^{®} can be applied to the renal artery as well, however, it must be removed before proceeding to the next step in the procedure. Alternatively, Composition 1 can be removed as described above by washing with saline.

Composition 1 also can be applied for preventing bleeding, so stripping can be performed in an efficient and uninterrupted manner. Composition 1 is a transparent material, even after it adopts a gelled state, thus it does not hinder the identification of the location of the tumor. By the advance application of Composition 1, exudative bleeding can be prevented and maintain a clear surgical field for the surgeon. Further, Composition 1 decreases the requirement for clamping the renal vein.

Application of Composition 1 in advance or instantly to an oozing bleeding site will attain arrest of bleeding and prevent further bleeding, all of which contributes to a clear view of the surgical field. Surgical procedure time is decreased by the substitution of Composition 1 for traditional, and often complicated, bleeding control measures such as SURGICEL^{®} and fibrin glue. After the removal of extra Composition 1 using a saline wash, prevention of bleeding can be confirmed easily. Further, by applying Composition 1 to the circumference after the suture of the renal calyx, post-bleeding prevention is achieved.

Less than 30 minutes of ischemic time is desire. In a temporary arrest of bleeding, fibrin glue and SURGICEL^{®} lead to obstruction of the surgical field of view and extend surgical time as it is necessary for them to be removed. Composition 1 has an advantage of being able to decrease the overall time required to complete the procedure by its use throughout the procedure, all the while maintaining a clear surgical field of view due to its transparent quality.

If bleeding cannot be controlled during laparoscopy, the surgeon must change to open surgery. However, prevention of exudative bleeding by application of Composition 1 is likely to reduce this risk significantly. It is difficult to arrest bleeding by gauze astriction in laparoscopy, so application of Composition 1 is uniquely suited for this surgical procedure. Due to the possibilities of bleeding partial renal resection after laparoscopy (three to eight percent), the need for reliable measures to control bleeding are high.

### Example 14. Clinical study of application of Composition 1 in multiple surgical procedures

The present Example describes various intrabody surgical procedures utilizing peptide compositions described herein and, in particular, steps of the surgical method at which peptide compositions described herein can be applied.

In particular, Hemorrhages in various surgical and endoscopic procedures (especially exudative hemorrhages) were designated as target sites for application of Composition 1. The primary endpoint was intraoperative bleeding, while the secondary endpoint was postoperative bleeding. Other objectives of the study included verification of safety (occurrence of adverse events). The protocol established for this study was an open-label, non-controlled, multicenter collaborative study, which was approved by the company Institutional Review Board (IRB) and communicated to the regulatory authority, Pharmaceuticals and Medical Devices Agency (PMDA). Once the protocol approved by the PMDA was discussed and approved by the IRB of each study facility, the study was initiated.

Composition 1 was manufactured by dissolving the starting peptide, CH₃CO-(Arg-Ala-Asp-Ala)₄-NH₂, in water for injection at a concentration of 2.5% (w/v). Syringes were pre-filled in an aseptic manner and packaged into blister-packaging. The exterior of both packaging and the syringes were sterilized with ethylene oxide.

In this study, direct application and application of Composition 1 via transcatheter was employed. In the cases of direct application, a plastic disposable nozzle was attached to a syringe and used to apply Composition 1 to the bleeding site. In the cases of transcatheter application, a catheter was attached to a syringe tip and applied to the bleeding site, with visual confirmation by monitor.

The target surgical procedures chosen for this clinical study were endoscopic mucosal resection (EMR), endoscopic sub mucosal dissection (ESD), angiostomy (vessel-to-vessel anastomosis or vessel-to-artificial vessel anastomosis in coronary bypass or other vascular surgery), and hepatectomy (hepatic lobectomy, hepatic segmentectomy or partial liver resection including laparoscopic hepatectomy and laparoscopically assisted hepatectomy). The hemorrhages targeted for application of Composition exudative hemorrhages, and the surgeon determined by visual inspection if a hemorrhage was within this purview. The target patients were inpatients or scheduled in patients who were scheduled to undergo one of these surgical procedures. Patients (between the ages of 20 - 80) were given an explanation of written consent prior to surgery and required to provide written consent before surgery.

Approximately 10 patients per facility and approximately 100 patients in total were set as a target enrollment for the study. Assuming 100 as the target number of patients and 85% efficacy of Composition 1 expected based on the results observed in animals, estimated accuracy is 7%, and the lower limit of the estimate range for the rate of complete arrest of bleeding rate by Composition 1 can be calculated as 78% [(expected efficacy * (1-expected hemostatic efficacy)/(estimate accuracy/1.96)²=0.85(1-0.85) / (0.07 / 1.96)² = 99.9)]. Consequently, observation of about 100 cases was believed to allow determination of whether the efficacy rate of Composition 1 could exceed the 76.9% mean complete arrest of bleeding rate for exudative hemorrhage in studies of previously-approved materials (AVITENE^{®}, INTEGRAN^{®}, BOLHEAL^{®}, and TACHOCOMB^{®}).

The study was conducted as an open-label, non-controlled study at 10 facilities (two facilities performing endoscopic surgery of the upper GI tract, four facilities performing cardiovascular surgery, and four facilities performing gastrointestinal surgery). The study was divided into an investigational phase and a validation phase; the Independent Data Monitoring Committee (IDMC) performed an interim review of the first three cases in the feasibility phase, and in the pivotal phase, the study was continued until the target number of enrolled patients was reached.

The primary endpoint for efficacy was occurrence of complete stoppage of bleeding upon application of Composition 1 and intraoperative maintenance of the same in exudative hemorrhages suitable for application of Composition 1 without use of standard means such as ligation or cauterization. Hemorrhages were excluded from the target sites of the material application in the study if they were heavier than exudative hemorrhages and the first choice treatment would usually be ligation, cauterization, or other such means.

In endoscopic surgery, the Composition 1 was applied to hemorrhages occurring during resection or dissection of the involved site in standard EMR or ESD, after the surgeon visually determined whether the hemorrhage was an exudative hemorrhage suitable for application with Composition 1. Likewise, in angiostomy, Composition 1 was applied to hemorrhages occurring at vascular anastomosis sites when blood flow was restarted after standard angiostomy, after the surgeon visually determined whether the hemorrhage was an exudative hemorrhage suitable for application with Composition 1. In hepatectomy as well, Composition 1 was applied after the surgeon determined visually whether a hemorrhage occurring during or after standard hepatectomy (including open and laparoscopic surgeries) was an exudative hemorrhage suitable for application with Composition 1. Visual inspection by the surgeon to determine if application of Composition 1 had achieved the endpoints at the application site(s). If needed, video or photographic imaging data that could be obtained was used to aid confirmation and evaluation of effect.

The secondary endpoint for evaluation of efficacy was occurrence of secondary hemorrhage on postoperative Day 1 and on postoperative Days 5 to 7 (if a patient was discharged before Day 5, the day preceding discharge or the discharge day); this endpoint ascertained postoperative maintenance at the application site of Composition 1 application.

In endoscopic surgery, occurrence of secondary hemorrhage was confirmed directly by endoscopic examination on postoperative Day 1, and a second evaluation for secondary hemorrhage was performed by blood tests. In angiostomy, occurrence of secondary hemorrhage was ascertained on postoperative Day 1 by the color of exudate in a drain for patients with a drain, and by blood testing for patients without a drain; blood testing was also used for a second evaluation of secondary hemorrhage. In hepatectomy, occurrence of secondary hemorrhage was ascertained by the color of exudate in a drain in postoperative Day 1, and blood testing was used for a second evaluation of secondary hemorrhage.

As a safety endpoint, all problems and/or adverse events (including abnormal changes in laboratory test results) occurring during the patient observation period were evaluated in causal relationship with Composition 1 and the study. Patients were treated promptly as needed.

Other endpoints included: operation time (distribution, mean value, and standard error of bleeding time were calculated for application sites allowing measurement of time from the point of application to evaluation of complete arrest of bleeding) and operability (difference in the ease of use versus existing materials and drugs was expressed numerically as follows: Excellent = 3, Good = 2, Acceptable = 1 and Unacceptable = 0; frequency distribution, mean and standard deviation of the assessment results were calculated).

Safety and efficacy analyses were conducted on a safety analysis set (SAS) and full analysis set (FAS) respectively. The SAS consisted of all subjects to which Composition 1 was applied. The efficacy analysis set was the FAS, defined to exclude subjects enrolled in the study who contravened inclusion criteria. Because the study included patients to whom Composition 1 was applied at multiple points, data were tabulated and analyzed for each hemorrhage site. At some hemorrhage sites, application was deemed inappropriate according to the study the protocol; these were excluded from the FAS and also analyzed as a per protocol set (PPS). Each patient was also evaluated for postoperative secondary hemorrhage, and some patients receiving postoperative treatment which may have affected secondary hemorrhage evaluation were also excluded from the FAS and analyzed in the PPS. The validity of the PPS designations was confirmed by the IDMC.

The study was conducted in accordance with the ethical principles based on the "World Medical Association Declaration of Helsinki" (drafted by the 18th WMA General Assembly in Helsinki, 1964; revised by the 55th General Assembly in Tokyo, October 2004; latest revision by the 59th General Assembly in Seoul, October 2008), to which all medical research involving human subjects must conform, and in compliance with the Pharmaceutical Affairs Law and standards of Medical Device Good Clinical Practice (GCP Ministerial Ordinance).

The results were analyzed by efficacy endpoints, safety endpoints and other endpoints as described above.

**Efficacy: primary endpoint (Table** 5). The results demonstrated that the efficacy rate of stopping bleeding for Composition 1 in the FAS for all three target surgical procedures combined was 82.5% (160/194 sites), and the efficacy rate in the PPS for all three target surgical procedures combined was 88.8% (158/178 sites).

Efficacy: secondary endpoint. The results demonstrated that the efficacy rate of stopping bleeding for secondary hemorrhage with Composition 1 in the FAS, for all three target surgical procedures combined, was 100.0% (89/89 patients) both on postoperative Day 1 and on postoperative Days 5 to 7. The efficacy rate in the PPS for all three target surgical procedures combined was 100.0% (79/79 patients) on postoperative Day 1 and 100.0% (78/78 patients) on postoperative Days 5 to 7.

Safety (Table 6). During the study period, no occurrence of problems, e.g., product failure, was observed at a study facility. During the observation phase of the study, 53 adverse events occurred among the 97 patients in the SAS, and a causal relationship with Composition 1 could not be disqualified for three adverse events. Of these three adverse events, two were abnormal laboratory test results (elevated test values related to liver function (AST, ALT, Al-P) and elevated uric acid), but their degree of abnormality was deemed clinically insignificant. The remaining adverse event was discoloration of an artificial vessel, which the Principal Investigator deemed clinically insignificant. The adverse events which occurred during the observation phase of the study were principally associated with surgical invasion, and after symptoms were confirmed, adverse events requiring treatment were treated appropriately.

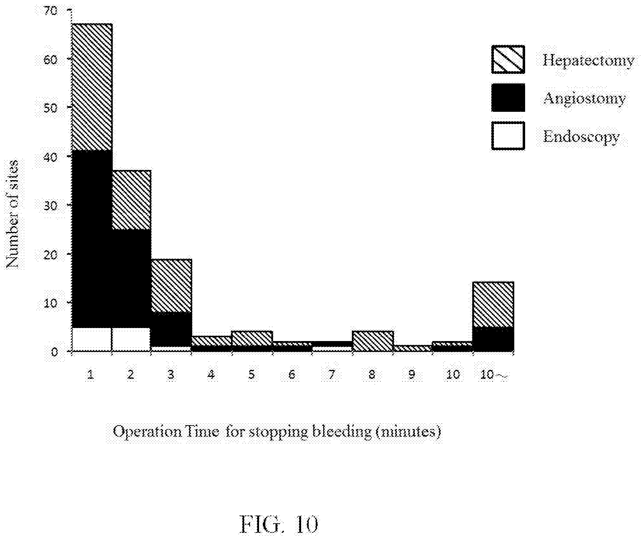

**Operation time and Operability (****Figure 10****).** The mean value of operation time relating to stoppage of bleeding when using Composition 1 at 155 sites was about four minutes and 42 seconds (0:04:42). At most sites, complete arrest of bleeding was achieved in less than three minutes. The mean value of Composition 1 operability for all 96 FAS patients was 2.4, and in most cases, operability was evaluated as better than existing materials (Table 7).

**TABLE 5**

| Surgical Method | Extremely Effective | Effective | Somewhat Effective | Ineffective | Appl. Sites | Efficacy | 95% Confidence Interval |
|---|---|---|---|---|---|---|---|
| Endoscopic Operation | 11 | 1 | 0 | 0 | 12 | 100.0% (12/12/) | 73.5%-100.0% |
| Angiostomy | 66 | 8 | 2 | 1 | 77 | 96.1% (74/77) | 89.0-99.2% |
| Hepatectomy | 72 | 0 | 3 | 14 | 89 | 80.9% (72/89) | 71.2-88.5% |
| Total | 149 | 9 | 5 | 15 | 178 | 88.8% (158/178) | 83.2-93.0% |

**TABLE 6**

| Adverse Event | Causal Relation | | |
|---|---|---|---|
| | Present | Not present | Total |
| Post-operative pain | | 9 (9.3%) | 9 (9.3%) |
| Vomiting | | 4 (4.1%) | 4 (4.1%) |
| Onset of Fever | | 4 (4.1%) | 4 (4.1%) |
| Nausea | | 3 (3.1%) | 3 (3.1%) |
| CRP Increase | | 3 (3.1%) | 3 (3.1%) |
| Ketone Bodies in Urine | | 2 (2.1%) | 2 (2.1%) |
| Edema | | 2 (2.1%) | 2 (2.1%) |
| Fibrinogen Score Increase | | 2 (2.1%) | 2 (2.1%) |
| Blood Count Abnormality | | 2 (2.1%) | 2 (2.1%) |
| Diarrhea | | 1 (1.0%) | 1 (1.0%) |
| ALBUMIN Score Decline | | 1 (1.0%) | 1 (1.0%) |
| Protein Abnormality | | 1 (1.0%) | 1 (1.0%) |
| S-FDP Increase | | 1 (1.0%) | 1 (1.0%) |
| ZT T Value Decrease | | 1 (1.0%) | 1 (1.0%) |
| Upset Stomach | | 1 (1.0%) | 1 (1.0%) |
| Liver Function Decrease | 1 (1.0%) | | 1 (1.0%) |
| High Blood Pressure | | 1 (1.0%) | 1 (1.0%) |
| Laryngopharynx Discomfort | | 1 (1.0%) | 1 (1.0%) |
| Hemorrhagic Shock | | 1 (1.0%) | 1 (1.0%) |
| Cardiac Tamponade | | 1 (1.0%) | 1 (1.0%) |
| Discoloration of Artificial Vessel | 1 (1.0%) | | 1 (1.0%) |
| Uric Acid Level Increase | 1 (1.0%) | | 1 (1.0%) |
| Increase in Total Bilirubin | | 1 (1.0%) | 1 (1.0%) |
| Hypokalemia | | 1 (1.0%) | 1 (1.0%) |
| Urine sugar | | 1 (1.0%) | 1 (1.0%) |
| Anemia | | 1 (1.0%) | 1 (1.0%) |
| Ascitic | | 1 (1.0%) | 1 (1.0%) |
| Poor Peripheral Circulation | | 1 (1.0%) | 1 (1.0%) |
| Lightheadedness | | 1 (1.0%) | 1 (1.0%) |
| Lumbar Pain | | 1 (1.0%) | 1 (1.0%) |
| Advanced Heart Muscle Edema | | 1 (1.0%) | 1 (1.0%) |
| Total | 3 (3.1%) | 50 (51.5%) | 53 (54.6%) |

**TABLE 7**

| Assessment (Evaluation Points) | Operation Type | | | |
|---|---|---|---|---|
| | Endoscopy | Angiostomy | Hepatectomy | Total |
| Excellent (3) | 2 | 37 | 11 | 50 |
| Good (2) | 8 | 7 | 16 | 31 |
| Acceptable (1) | 2 | 2 | 11 | 15 |
| Unacceptable (0) | 0 | 0 | 0 | 0 |
| Applicable No. of Subjects | 12 | 46 | 38 | 96 |
| Average Rating Points | 2.0 | 2.8 | 2.0 | 2.4 |
| Standard Deviation | 0.6 | 0.5 | 0.8 | 0.7 |

This study represents the first clinical evaluation of Composition 1 in various surgical settings. The efficacy rate of Composition 1 for arrest of bleeding at a surgical site was 82.5% (160/194 sites) in the FAS. The efficacy may be higher due to the inclusion of some application sites judged unsuitable according to the application procedure specified by the study protocol. In analysis of the PPS, from which these application sites were excluded, the efficacy rate was 88.8% (158/178 sites), which exceeds the 85% target efficacy rate of the study.

As shown in this example, the application of Composition 1 provides an effective alternative against exudative hemorrhage following each of the studied surgical procedures. Although a lower trend in the efficacy rate was demonstrated in hepatectomy, the result was nevertheless clinically effective. The range of bleeding sites targeted for treatment was wider in hepatectomy, and the number of hemorrhaging points at each site was greater than in other surgical procedures, indicating that the application of Composition 1 in hepatectomy represents a more difficult surgical situation as compared to other surgical procedures. In the early period of the study, some cases of hepatectomy presented sites where an appropriate application method could not be applied. Thus, hepatectomy may require additional training with respect to application of Composition 1.

Among the 97 patients in the safety analysis set, 53 adverse events occurred during the observation phase of the study. Of these, a causal relationship with Composition 1 could not be disqualified for three adverse events. Two of these events were abnormal laboratory test results and deemed not clinically significant. The remaining adverse event was discoloration of an artificial vessel, also deemed not clinically significant. Four serious adverse events were observed, however, not correlated to application of Composition 1. Overall, no Composition 1-related problems were noted during the study, which demonstrates that Composition is safe and effective for use on or at a surgical site during various surgical procedures.

Taken together, these data demonstrate that peptide compositions provided by the present invention can effectively inhibit bleeding encountered during various surgical methods, and, in particular, provide an advantage over existing methodology by providing improved efficacy and decreasing operation time.

The foregoing description and drawings are by way of example only and the invention is described in detail by the claims that follow.

### Equivalents

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

The articles "a" and "an" as used herein in the specification and in the claims, unless clearly indicated to the contrary, should be understood to include the plural referents. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or the entire group members are present in, employed in, or otherwise relevant to a given product or process. Where elements are presented as lists, (e.g., in Markush group or similar format) it is to be understood that each subgroup of the elements is also disclosed, and any element(s) can be removed from the group. It should be understood that, in general, where the invention, or aspects of the invention, is/are referred to as comprising particular elements, features, etc., certain embodiments of the invention or aspects of the invention consist, or consist essentially of, such elements, features, etc. For purposes of simplicity those embodiments have not in every case been specifically set forth in so many words herein. The publications, websites and other reference materials referenced herein describe the background of the invention and provide additional detail regarding its practice.

## Claims

1. A composition, comprising:
a peptide solution comprising about 1% to about 3% (w/v) of a peptide, wherein the peptide has an amino acid sequence of n-RADARADARADARADA-c; and wherein the solution is **characterized by** an ability to transition between two states: an ungelled state adopted when one or more particular ions is substantially absent, and a gelled state adopted when the one or more ions is present at or above a threshold level, wherein the one or more ions is or becomes present, for use in a method of preventing post-operative bleeding after a surgical procedure performed on a human patient comprising application of the composition to a bleeding surgical site, wherein the patient is dosed with an anticoagulant.

2. The composition for use according to claim 1, wherein the method comprises administering protamine sulfate to the patient after the surgical procedure.

3. The composition for use according to claim 1, wherein the surgical procedure is selected from the group consisting of coronary artery bypass graft (CABG), vascular surgery for abdominal aortic aneurism (AAA), and arteriosclerosis obliterans (ASO).

4. The composition for use according to claim 3, wherein the use comprises removing the composition from the site by washing the treated surgical site with a saline wash.

5. The composition for use according to any one of claims 1-4, wherein the use comprises one or more additional applications of the composition to the surgical site.

6. The composition for use according to any one of claims 1-5, wherein the one or more ions are potassium (K⁺) and sodium (Na⁺).

## Patentansprüche

1. Zusammensetzung, umfassend:
eine Peptidlösung, die zu etwa 1 % bis etwa 3 % (w/v) ein Peptid umfasst, wobei das Peptid die Aminosäuresequenz n-RADARADARADARADA-c aufweist; und wobei die Lösung **gekennzeichnet ist durch** eine Fähigkeit, zwischen zwei Zuständen zu wechseln: einem nicht gelierten Zustand, der eingenommen wird, wenn ein oder mehrere bestimmte Ionen im Wesentlichen fehlen, und einem gelierten Zustand, der eingenommen wird, wenn das eine oder die mehreren Ionen bei oder über einem Schwellenwert vorhanden sind, wobei das eine oder die mehreren Ionen vorhanden sind oder werden, zur Verwendung in einem Verfahren zum Verhindern postoperativer Blutungen nach einem chirurgischen Eingriff an einem menschlichen Patienten, umfassend das Auftragen der Zusammensetzung auf eine blutende Operationsstelle, wobei dem Patienten ein Antikoagulans zudosiert wird.

2. Zusammensetzung zur Verwendung nach Anspruch 1, wobei das Verfahren das Verabreichen von Protaminsulfat an den Patienten nach dem chirurgischen Eingriff umfasst.

3. Zusammensetzung zur Verwendung nach Anspruch 1, wobei der chirurgische Eingriff ausgewählt ist aus der Gruppe bestehend aus Koronararterien-Bypass (CABG), Gefäßchirurgie bei abdominalem Aortenaneurysma (AAA) und Arteriosklerose obliterans (ASO).

4. Zusammensetzung zur Verwendung nach Anspruch 3, wobei die Verwendung das Entfernen der Zusammensetzung von der Stelle durch Waschen der behandelten Operationsstelle mit einer Kochsalzlösung umfasst.

5. Zusammensetzung zur Verwendung nach einem der Ansprüche 1 bis 4, wobei die Verwendung eine oder mehrere zusätzliche Anwendungen der Zusammensetzung an der Operationsstelle umfasst.

6. Zusammensetzung zur Verwendung nach einem der Ansprüche 1 bis 5, wobei das eine oder die mehreren Ionen Kalium (K⁺) und Natrium (Na⁺) sind.

## Revendications

1. Composition comprenant :
une solution de peptide comprenant environ 1 % à environ 3 % (p/v) d'un peptide, dans laquelle le peptide a une séquence d'acides aminés de n-RADARADARADARADA-c ; et dans laquelle la solution est **caractérisée par** une capacité de transition entre deux états : un état non gélifié adopté lorsqu'un ou plusieurs ions particuliers sont pratiquement absents, et un état gélifié adopté lorsque le ou les ions sont présents à un niveau supérieur ou égal à un seuil, dans laquelle le ou les ions sont ou deviennent présents, pour une utilisation dans un procédé de prévention des saignements post-opératoires après une procédure chirurgicale effectuée sur un patient humain comprenant l'application de la composition sur un site chirurgical qui saigne, dans lequel le patient reçoit une dose d'anticoagulant.

2. Composition pour utilisation selon la revendication 1, dans laquelle le procédé comprend l'administration du sulfate de protamine au patient après l'intervention chirurgicale.

3. Composition pour utilisation selon la revendication 1, dans laquelle l'intervention chirurgicale est choisie dans le groupe constitué de pontage aorto-coronarien (PAC), chirurgie vasculaire pour l'anévrisme de l'aorte abdominale (AAA) et artériosclérose oblitérante (ASO).

4. Composition pour utilisation selon la revendication 3, dans laquelle l'utilisation comprend le retrait de la composition du site en lavant le site chirurgical traité avec une solution saline.

5. Composition pour utilisation selon l'une quelconque des revendications 1 à 4, dans laquelle l'utilisation comprend une ou plusieurs applications supplémentaires de la composition sur le site chirurgical.

6. Composition pour utilisation selon l'une quelconque des revendications 1 à 5, dans laquelle le ou les ions sont le potassium (K⁺) et le sodium (Na⁺).
